(19) Europäisches Patentamt
European Patent Office
Office européen des brevets

(11) **EP 2 528 567 B1**

(12) **EUROPÄISCHE PATENTSCHRIFT**

(45) Veröffentlichungstag und Bekanntmachung des Hinweises auf die Patenterteilung:
**16.09.2020 Patentblatt 2020/38**

(21) Anmeldenummer: **11705432.0**

(22) Anmeldetag: **28.01.2011**

(51) Int Cl.:
**A61F 13/537** (2006.01)      **A61F 13/53** (2006.01)
**A61F 13/534** (2006.01)      **A61F 13/15** (2006.01)

(86) Internationale Anmeldenummer:
**PCT/EP2011/000391**

(87) Internationale Veröffentlichungsnummer:
**WO 2011/092025 (04.08.2011 Gazette 2011/31)**

(54) **FLEXIBLES, STARK ABSORBIERENDES MATERIAL**

FLEXIBLE, HIGHLY ABSORBENT MATERIAL

MATÉRIAU SOUPLE ET FORTEMENT ABSORBANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.01.2010 US 657982**
**28.01.2010 DE 102010006228**

(43) Veröffentlichungstag der Anmeldung:
**05.12.2012 Patentblatt 2012/49**

(73) Patentinhaber: **Glatfelter Falkenhagen GmbH**
**16928 Pritzwalk (DE)**

(72) Erfinder:
• **RÖTTGER, Henning**
**24568 Kaltenkirchen (DE)**
• **LUTTER, Stefanie**
**16909 Wittstock (DE)**
• **EHMKE, Ralf**
**16945 Meyenburg (DE)**

(74) Vertreter: **Eisenführ Speiser**
**Patentanwälte Rechtsanwälte PartGmbB**
**Anna-Louisa-Karsch-Strasse 2**
**10178 Berlin (DE)**

(56) Entgegenhaltungen:
**EP-A1- 2 140 844        US-A1- 2002 165 509**
**US-A1- 2004 122 394**

**Beschreibung**

Die vorliegende Erfindung betrifft eine mehrlagige absorbierende Struktur mit zumindest einer Flüssigkeitsspeicherschicht, bevorzugt mit einer Flüssigkeitsaufnahmeschicht, einer Flüssigkeitsspeicherschicht und einer Flüssigkeitsverteilungsschicht, unter Verwendung von Cellulosefasern und superabsorbierendem Polymer, sowie ein Verfahren und eine Vorrichtung zur Herstellung der Struktur.

Mehrlagige absorbierende Strukturen unter Verwendung von Cellulose und superabsorbierenden Polymeren, abgekürzt SAP, beispielsweise in der Form von SAP-Partikel oder SAP-Fasern, sind seit Jahren bekannt und werden als Lagenmaterial in Wegwerfprodukten, beispielsweise in Hygieneprodukten, Medikalprodukten und industriellen Produkten genutzt.

Bekannt sind Faserbahnen mit saugfähigen Lagen, in denen Bindemittel zur Stabilisierung der saugfähigen Lage und zur Vermeidung des Abriebs der Fasern eingesetzt werden. Die Lagen eines derartigen Lagenmaterials enthalten ein Bindemittel, beispielsweise in Form von Fasern, Pulver, Hotmelt-Klebern, Lösungsmittel-Bindemittel-Gemischen, als Auftrag in flüssiger Form und ähnlichem.

Beispielsweise wird in EP 1 721 036 die Herstellung einer Faserbahn aus Cellulosefasern mit absorbierenden Eigenschaften in einem Airlaid-Verfahren beschrieben. Die Faserbahn weist mehrere Lagen auf. Eine innere saugfähige Lage enthält ein punktgebundenes Cellulosematerial mit einem Superabsorber. Zur Verhinderung des Faserstaubens, "linting" genannt, der durch Abrieb beziehungsweise Fusseln von Cellulosefasern entsteht, werden die Fasern insbesondere in den Außenbereichen der mittleren Faserbahn mit einem Wasser-Latex-Gemisch imprägniert.

Aus US 2002/0165509 A1 ist eine absorbierende Struktur bekannt, die eine Fluidaufnahmeschicht, eine Fluidverteilungsschicht und eine Fluidspeicherschicht aufweist. Die Fluidspeicherschicht ist zwischen der Fluidaufnahmeschicht und der Fluidverteilungsschicht angeordnet. Die Schichten sind miteinander in Fluidkommunikation.

Aus EP 2 140 844 A1 ist eine mehrlagige faserförmige Vliesstruktur bekannt. Die Struktur enthält eine erste Lage mit einer Mischung aus Cellulosefasern und synthetischen Fasern, eine zweite Lage mit einer Mischung aus Cellulosefasern und einem superabsorbierenden Pulver und einer dritten Lage, in der nur Cellulosefasern vorhanden sind.

Ferner ist aus US 2004/0122394 A1 ein absorbierender Kern für die Verwendung in einem absorbierenden Produkt, wie beispielsweise einer Windel oder einem Inkontinenzprodukt, bekannt. Der absorbierende Kern enthält eine erste stabilisierte absorbierende Schicht und eine zweite absorbierende Schicht die superabsorbierende und absorbierende Fasern enthält, die mit einem nicht-flüchtigen Verdichtungswirkstoff behandelt wurden.

Wird eine absorbierende Struktur, welches zum Beispiel eine Flüssigkeitsaufnahmeschicht, eine Flüssigkeitsspeicherschicht und eine Flüssigkeitsverteilungsschicht aufweist, von einer Flüssigkeit benetzt, gelangt die Flüssigkeit durch Kapillareffekte von der Flüssigkeitsaufnahmeschicht in die Flüssigkeitsspeicherschicht. Überschussige Flüssigkeit kann von der Flüssigkeitsspeicherschicht in die Flüssigkeitsverteilungsschicht eintreten und von dieser durch eine entsprechende Wahl der Porenstruktur verteilt werden, gegebenenfalls in die Speicherschicht zurückgeführt werden. Hierdurch können zwar unerwünschte Rücknässungseffekte, die durch ein Auslaufen von Flüssigkeit aus einer Flüssigkeitsaufnahmeschicht in eine Flüssigkeitsverteilungsschicht und über diese hinaus, beispielsweise auf die Haut eines Trägers eines Hygieneprodukts auftreten können, vermindert werden: Allerdings weisen derartige Produkte mitunter beträchtliche Defizite im Tragekomfort auf. So hat beispielsweise eine durch Flüssigkeitsaufnahme und anschließende Deformation, das heißt eine durch den Gebrauch befeuchtete und zusammengedrückte Flüssigkeitsspeicherschicht nach der Entlastung nicht das Bestreben, in die ursprüngliche Lage zurückzugehen. Bei Befeuchtung von beispielsweise SAP-Partikeln und Deformierung dieser kann ein Klebeffekt zwischen den Partikeln entstehen. Bei bekannten relativ kompakten Flüssigkeitsspeicherschichten wird hierdurch die Kapazität zur Aufnahme weiterer Flüssigkeit stark gemindert sein, was den Tragekomfort beeinträchtigt.

Es wäre daher wünschenswert, ein Produkt zur Verfügung zu haben, mit welchem derartige Einbussen des Tragekomforts im Gebrauch vermieden werden können.

Es ist daher Aufgabe der vorliegenden Erfindung, ein Produkt, ein Verfahren und eine Vorrichtung zur Herstellung eines absorbierenden Produktes zur Verfügung zu stellen, mit welchem ein besseres Verhalten während der Nutzung ermöglicht, insbesondere eine höhere Flexibilität ermöglicht.

Diese Aufgabe wird mit einer absorbierenden Struktur nach Anspruch 1, einem Verfahren zur Herstellung einer absorbierenden Struktur nach Anspruch 8 und einer Vorrichtung nach Anspruch 9 gelöst. Bevorzugte Ausführungsformen sind in den Unteransprüchen definiert. Die darin enthaltenen Merkmale sind jedoch auch mit anderen Merkmalen aus der nachfolgenden Beschreibung zu weiteren Ausgestaltungen verknüpfbar und nicht allein auf die jeweilige beanspruchte Weiterbildung beschränkt. Auch dienen die vorgeschlagenen jeweiligen Merkmale insbesondere auch der jeweiligen unabhängigen Ansprüche nur als erster Vorschlag einer Lösung, wobei ein oder mehrere der in den unabhängigen Ansprüchen enthaltenen Merkmale auch durch die nachfolgenden Merkmale ergänzt und/oder ausgetauscht werden können.

Es wird eine absorbierende Struktur mit einer Folge an Schichten umfassend eine erste und eine zweite Au-

ßenoberfläche, eine erste flüssigkeitsspeichernde Schicht, eine zweite flüssigkeitsspeichernde Schicht und mindestens eine zwischen der ersten flüssigkeitsspeichernden Schicht und der zweiten flüssigkeitsspeichernden Schicht und im direkten Kontakt mit diesen angeordnete Flüssigkeitsspeicherschicht vorgeschlagen, wobei die Schichten übereinanderliegen und eine Lagenstruktur bilden. Die erste flüssigkeitsspeichernde Schicht enthält Fluffpulp, Bindefasern und superabsorbierendes Polymer. Die zweite flüssigkeitsspeichernde Schicht enthält Fluffpulp und Bindefasern. Die zumindest eine Flüssigkeitsspeicherschicht weist ein Fluffpulp, ein superabsorbierendes Polymer SAP, vorzugsweise SAP-Partikel und/oder SAP-Fasern auf, wobei die Flussigkeitsspeicherschicht kein Bindemittel aufweist. Wenigstens eine der ersten und zweiten Außenoberflächen weist eine Latex-basierte Bindemittelschicht auf.

Zur Flüssigkeitsspeicherschicht benachbarte, flüssigkeitspeichernde Lagen der absorbierenden Struktur können gemäß einer Ausgestaltung auch zumindest eine derjenigen Lagen umfassen, die die erste und die zweite Außenoberfläche der absorbierenden Struktur bilden.

In einer bevorzugten Ausführungsform weist die absorbierende Struktur mindestens eine Flüssigkeitsaufnahmeschicht, mindestens eine nachfolgende Flüssigkeitsspeicherschicht und mindestens eine nachfolgende Flüssigkeitsverteilungsschicht auf, wobei die Schichten verbunden sind und eine Lagenstruktur bilden. Die Flüssigkeitsaufnahmeschicht und/oder die Flüssigkeitsspeicherschicht weisen ein Cellulosematerial, vorzugsweise Cellulosefasern und ein superabsorbierendes Polymer SAP, vorzugsweise SAP-Partikel und/oder SAP-Fasern auf. Die Flüssigkeitsaufnahmeschicht und/oder die Flüssigkeitsverteilungsschicht beinhalten ein Bindemittel. Im Gegensatz zu den Cellulosefasern der Flüssigkeitsaufnahmeschicht und der Flüssigkeitsverteilungsschicht sind die Cellulosefasern der Flüssigkeitsspeicherschicht nicht mit einem Bindemittel imprägniert beziehungsweise weisen kein Bindemittel auf. Bei Flüssigkeitsaufnahme können die Haftverbindungen sich lösen und erlauben eine Beweglichkeit innerhalb der Flüssigkeitsspeicherlage. Auch kann bei einer Nutzung der absorbierenden Struktur eine Scherkraft auftreten, die sodann die Haftverbindungen auflöst und die Beweglichkeit in der Flüssigkeitsspeicherlage herstellt.

Bevorzugt weist die Lagenstruktur gemäß einer Ausgestaltung zumindest folgenden Aufbau auf:

- eine erste Lage, umfassend zumindest einen behandelten Fluffpulp, Mehrkomponentenfasern, vorzugsweise PE/PET-Bikomponentenfasern und einen Superabsorber,

- eine zweite Lage, umfassend zumindest einen behandelten und/oder unbehandelten Fluffpulp und einen Superabsorber und

- eine dritte Lage, umfassend zumindest einen unbehandelten Fluffpulp und Mehrkomponentenfasern, vorzugsweise PE/PET-Bikomponentenfasern.

Zur Unterstützung der ersten Lage kann an deren äußerer Oberfläche zudem eine Papier-Tissue-Lage vorgesehen sein. In einer Ausführungsform weist diese Lagenstruktur eine lockere Verdichtung durch einen Kalandrierschritt, vorzugsweise mittels eines Glattwalzenkalanders auf, wodurch eine erste Dicke, zum Beispiel eine Enddicke des Lagenmaterials einstellbar ist.

Außerdem ist ein Latex-Binder, beispielsweise ein EVA-Dispersionbinder auf einer oder beiden äußeren Oberflächen der Lagenstruktur aufgebracht, der anschließend ausgetrocknet und ausgehartet ist. Im Gegensatz zu herkömmlichen Materialien gestattet der vorgeschlagene Aufbau der Lagenstruktur, dass der Superabsorber in der Mittellage, die bevorzugt durch die Flüssigkeitsspeicherschicht gebildet oder zumindest mitgebildet wird, nach einem Flüssigkeitseintrag frei quellen kann, da er nicht durch Bindefasern gehemmt ist. Dieser Quellvorgang wird beispielsweise durch eine mit EVA-Dispersionsbinder versteifte, aber offene Pulp-Bico-Lage nicht behindert.

Bevorzugt wird hierdurch eine elastische Lagenstruktur zur Verfügung gestellt, die feste Außenlagen und zumindest eine lockere, kaum kompaktierte Innenlage aufweist.

Die Lagenstruktur ist auch ohne Tissue-Lage und/oder mit anderen Träger-Lagen, beispielsweise Spunbond-Lagen oder beliebig hergestellten Vliesen erzeugbar.

Das Lagenmaterial weist gemäß einer Ausgestaltung eine überwiegend gleichmäßige Dichte auf, die in einer Größenordnung von etwa $0,1$ $g/cm^3$ liegt. Sie kann auch in einem Bereich zwischen $0,08$ bis $0,15$ $g/cm^3$ liegen. Weiterhin kann gemäß einer Ausgestaltung im trockenen Zustand das Lagenmaterial bei einer Dicke von etwa 5 mm ein Flächengewicht von etwa 460 $g/m^2$ und bei einer Dicke von etwa 6 mm ein Flächengewicht von etwa 600 $g/m^2$ aufweisen. Ein Flächengewicht in einem Bereich zwischen 440 $g/m^2$ bis zu 680 $g/m^2$ wird angestrebt. Eine Dicke zwischen 4,7 mm und 6,7 mm der absorbierenden Struktur wird ebenfalls angestrebt. Hierbei handelt es sich um eine erzeugte Schichtdicke der Lagenstruktur, die diese im trockenen Zustand aufweisen kann. Neben diesen bevorzugten Schichtdicken sind für die erfindungsgemäße Lagenstruktur in Abhängigkeit von der jeweiligen Verwendung der Lagenstruktur und dem damit verbundenen Flüssigkeitsanfall beziehungsweise der Häufigkeit des Flüssigkeitsanfalls innerhalb eines kurzen Zeitintervall Schichtdicken zwischen 3 mm und 15 mm, vorzugsweise zwischen 4 mm und 8 mm denkbar. Bei Benutzung der Lagenstruktur, dass heißt im Nasszustand, wird sich die Schichtdicke auf Grund der enormen Aufnahmefähigkeit der Flüssigkeitsaufnahmeschicht und die relative Beweglichkeit der an ihr angrenzenden Schichten wesentlich geringer ändern, als dieses von herkömmlichen Produkten bekannt ist. Die Schichtdickenzunahme liegt je nach Sättigungsgrad im Bereich zwischen 50 % bis 150 %, bezogen auf die Ausgangsdicke der Lagenstruktur.

Überraschenderweise hat sich herausgestellt, dass das Lagenmaterial wesentliche Vorteile gegenüber üblichen aus dem Stand der Technik bekannten Fluff-Pulp-SAP-Lagenstrukturen oder Lagenstrukturen mit homogen thermisch gebundenen Schichten aufweist, in denen alle Schichten mit Bindern, beispielsweise mit Bikomponentenfasern ausgerüstet sind. Insbesondere im Zustand der Benutzung, also im nassen Zustand hat sich die vorgeschlagene Lagenstruktur als vorteilhaft erwiesen.

So hat sich beispielsweise gezeigt, dass die absorbierende Struktur den Vorteil aufweist, dass die Flüssigkeitsspeicherschicht während des Gebrauchs der absorbierenden Struktur trotz mehrfacher Flüssigkeitsaufnahme und Druckbelastung ihren ursprünglichen Zustand weitestgehend wieder einnehmen kann. Da keine Bindefasern vorhanden sind, wird das Superabsorbermaterial nicht am freien Quellen gehindert. Somit kann beispielsweise der gewünschte Parameter der freien Quellkapazität, die sogenannte Free Swell-Kapazität, der vorzugsweise im Bereich von 20 g/g liegt, mit vergleichsweise geringeren SAP-Anteilen und niedrigeren Flächengewichten erhalten werden, als dieses bei herkömmlichen Lagenstrukturen gegeben ist. Dadurch können Rohstoffkosten gesenkt werden. Gemäß einer Ausgestaltung liegt die freie Quellkapazität in einem Bereich zwischen 17 g/g und 24 g/g.

Zudem wurde bei bekannten Lagenstrukturen beobachtet, dass der Superabsorber nach einem Flüssigkeitseintrag wie ein Klebstoff wirkt und die Schicht in der sich ausgebildeten Form fixiert. Wenn das an sich lose Material deformiert und in dieser Form fixiert wird, hat es jetzt kein Bestreben mehr, seine ursprüngliche Form einzunehmen. Unterstützt durch die Schwerkraft bildet es Klumpen, das sogenannte "Sagging", wodurch der Tragekomfort und die Saugleistung sinken, da der Superabsorber nicht mehr optimal in der Flüssigkeitsspeicherschicht verteilt ist. Mit der vorgeschlagenen Lagenstruktur werden diese Nachteile überwunden, so dass ein Material mit geringerer Nasssteife, dass heißt einer größeren Beweglichkeit und einem hohen Tragekomfort bereitgestellt wird gegenüber dem jeweils gleichen Material, dass über ein Bindemittel innerhalb der Flüssigkeitsspeicherlage enthält.

Als vorteilhaft erweist sich zudem, dass das SAP-Material, welches bereits in der Flüssigkeitsaufnahmeschicht angeordnet ist, eintretende Flüssigkeit sofort aufnimmt und somit einen schnelleren Abtransport der Flüssigkeit von der Stelle ihrer Aufgabe zum Kern eines Absorberproduktes hin ermöglicht. Hierdurch werden Rücknässungseffekte, beispielsweise durch Auslaufen überschüssiger Flüssigkeit bei hohem Flüssigkeitsaufkommen, zum Träger eines Absorberproduktes hin, vermieden. Zudem wird beispielsweise eine Verbesserung der Wiederbenetzbarkeit der absorbierenden Struktur ermöglicht.

Die absorbierende Struktur weist mindestens drei Lagen auf, wobei eine mittlere Lage kein Bindemittel aufweist.

Vorgesehen ist ebenfalls, dass alle Schichten der Lagenstruktur jeweils mindestens ein Airlaid-Material aufweisen, vorzugsweise als Hauptbestandteil der Schicht, wobei die jeweiligen Schichten selbst mehrlagig ausgebildet sein können. Beispielsweise können die Flüssigkeitsaufnahmeschicht, die Flüssigkeitsspeicherschicht und/oder die Flüssigkeitsverteilungsschicht neben der Airlaid-Schicht ein oder mehrere funktionale Schichten aufweisen, die den gewünschten Effekt jeder dieser Schichten verstärken. Vorzugsweise können die einzelnen Schichten einer Lage derart angeordnet sein, dass von einer äußeren Oberfläche der Lage zur anderen äußeren Oberfläche hin ein Eigenschaftsgradient erzeugbar ist. Andererseits können die Schichten einer Lage derart angeordnet sein, dass innerhalb einer Lage ein Optimum der erwünschten Eigenschaften erzeugt wird.

Eine weitere Ausführung der Erfindung sieht vor, dass die mittlere Lage mit keinem Bindemittel zumindest zum überwiegenden Teil aus behandeltem und/oder unbehandeltem Cellulosematerial besteht.

Gemäß einer Weiterbildung ist das Bindemittel der Flüssigkeitsaufnahmeschicht und/oder der Flüssigkeitsverteilungsschicht als thermoplastische Fasern, vorzugsweise Mehrkomponentenfasern, besonders bevorzugt Bikomponentenfasern ausgebildet.

Nach einer weiteren Ausführung der Erfindung sind SAP-Partikel und/oder SAP-Fasern in der Flüssigkeitsspeicherschicht bei Flüssigkeitsaufnahme und/oder unter Druck beweglich zueinander angeordnet.

Die Lagenstruktur weist eine erste und eine zweite Oberfläche auf, wobei die erste und/oder die zweite Oberflache jeweils eine Latex-basierte Bindemittelschicht aufweist.

Gemäß einer Weiterbildung weist die absorbierende Struktur eine Paper Tissue Lage, eine zweite Lage mit Fluffpulp, Bindefasern und Superabsorber, eine dritte Lage mit Fluff-pulp ohne Bindefasern, und mit Superabsorber und eine vierte Lage mit Fluffpulp und Bindefasern auf.

Eine Weiterbildung der Erfindung sieht vor, dass die Flüssigkeitsaufnahmeschicht ein voluminöses Vlies aus behandeltem und/oder unbehandeltem Cellulosematerial aufweist.

Die einzelnen Lagen der absorbierenden Struktur können

- jeweils die gleiche Sorte an Cellulosefasern,

- jeweils unterschiedliche Sorten an Cellulosefasern,

- Mischungen hieraus,

- chemisch und/oder physikalisch behandelte Cellulosefasern, unbehandelte Cellulosefasern,

- Mischungen aus behandelten und unbehandelten Cellulosefasern,

- synthetische Fasern allein oder in Mischung mit Cellulosefasern in behandelter oder unbehandelter Form, sowie

- Fasern mineralischen Ursprungs allein oder in Mischung mit synthetischen und/oder Cellulosefasern

aufweisen. Einzelne Lagen können auch ausschließlich Cellulosefasern aufweisen.

Der Begriff "Cellulosefasern" wird im Rahmen der Offenbarung nicht einschränkend verstanden. Einsetzbar sind jegliche Naturfasern, die in der Lage sind oder durch eine chemisch und/oder physikalische Behandlung in die Lage versetzt worden sind, Flüssigkeiten aufzunehmen und vorzugsweise auch zu binden. Durch eine ebensolche Behandlung können auch synthetische Fasern und Fasern mineralischen Ursprungs aufbereitet sein.

Prinzipiell können alle Schichten der Lagenstruktur behandelte und/oder unbehandelte Cellulosefasern aufweisen. Es hat sich jedoch als zweckmäßig erwiesen, dass die Flüssigkeitsaufnahmeschicht eine Airlaid-Lage umfasst, die im Wesentlichen chemisch und/oder physikalisch unbehandelte Cellulosefasern aufweist. Gemäß einer weiteren Ausgestaltung sind die Cellulosefasern der Flüssigkeitsverteilungsschicht chemisch und/oder physikalisch behandelt.

Unter einer chemischen Behandlung werden beispielsweise

- Waschvorgänge, Extrahiervorgänge,
- Bleichvorgänge,
- Färbevorgänge,
- Fibrillierungsvorgänge unter Verwendung von Lösemitteln,
- Oberflächenbehandlung, vorzugsweise zur Hydrophilierung, Erhöhung der Festigkeit oder Elastizität, beispielsweise durch Sprühen, Tauchen, Tränken, Waschen

und Ähnliches verstanden.

Eine physikalische Behandlung kann durch

- Zerkleinerung und Fibrillierung, beispielsweise Schneiden, Mahlen, Zerfasern,
- Klassierung, beispielsweise Windsichten

erfolgen.

Je nach Art des Aufschluss-Prozesses für die Fasern und des Bleich-Prozesses sind definierte Eigenschaftskombinationen in den Cellulosefasern erreichbar.

Beispielsweise werden in der Flüssigkeitsspeicherlage vorzugsweise unbehandelte Fasern eingesetzt. Dies hat unterschiedliche Gründe. Der Pulp verliert durch das Zusetzen von Behandlungsmitteln, insbesondere Oberflächenbehandlungsmitteln an Absorptionsfähigkeit. Um in einer Flüssigkeitsspeicherlage eine bestmögliche Absorption zu gewährleisten, wird bevorzugt ein unbehandelter Pulptyp eingesetzt. Dieser lässt sich auch am besten im Prozess verdichten, da die unbehandelten Fasern gut zusammenhaften.

In der erfindungsgemäßen Lagenstruktur können vorteilhafterweise folgende Komponenten, jeweils bezogen auf das Gesamtgewicht der Lagenstruktur

- 2-10 Gew.-% eines Tissues, bevorzugt 3-4 Gew.-%,
- 20 - 60 Gew.-% Cellulosefasern in behandelter oder unbehandelter Form, vorzugsweise 35 - 45 Gew.-%, besonders bevorzugt jeweils 15-25 Gew.-% behandelte und unbehandelte Cellulose,
- 30 - 50 Gew.-% Superabsorber-Material, bevorzugt 40 - 45 Gew.-%,
- 1-5 Gew.-% eines ersten Bindemittels, vorzugsweise 3-4 Gew.-%, wobei das erste Bindemittel bevorzugt eine Polymerdispersion aufweist, besonders bevorzugt wird ein Latex-Binder,
- 3-10 Gew.-% eines zweiten Bindemittel, vorzugsweise 5-7 Gew.-%, wobei das zweite Bindemittel Mehrkomponentenfasern, vorzugsweise BikomponentenFasern auf der Basis von Polyethylen und Polyethylenterephthalat (PET) enthält,

angeordnet sein, wobei die angegebenen Bereiche der Komponenten anteilsmäßig auf die einzelnen Lagen der Struktur aufgeteilt sein können oder eine oder mehrere Komponenten in einer Lage nicht angeordnet sein können.

Wird die absorbierende Struktur einer Flüssigkeitsaufnahme und/oder einem leichten Druck ausgesetzt, so sind zumindest die SAP-Partikel und/oder SAP-Fasern der Flüssigkeitsspeicherschicht bei Flüssigkeitsaufnahme und/oder leichtem Druck in der Lage, ihre äußere Form weitestgehend beizubehalten. Bei dieser Belastung kommt es zu einer Quellung der SAP-Partikel und/oder SAP-Fasern und gegebenenfalls zu einer Deformation. Nach der Entlastung ist das SAP-Material in der Lage, seine Ausgangsform weitestgehend wieder anzunehmen.

Eine Ausgestaltung der absorbierende Struktur sieht vor, dass diese eine Paper Tissue Lage als erste Lage, eine zweite Lage mit Fluffpulp, Bindefasern und Superabsorber, eine dritte Lage mit Fluffpulp und mit Superabsorber und eine vierte Lage mit Fluffpulp und Bindefasern aufweist, wobei die dritte Lage ohne Bindefasern ist. Gemäß einer Weiterbildung ist vorgesehen, dass in einem nassen Zustand der absorbierenden Struktur die dritte Lage eine relative Beweglichkeit zwischen der zweiten und der vierten Lage in einer Längsrichtung der absorbierenden Struktur ermöglicht.

Das superabsorbierende Material, wie zum Beispiel in Form von oben schon dargestellten SAP-Partikeln und/oder SAP-Fasern, ist schwellfähig und geht in der Regel in einen gelartigen Zustand über. Sie können dadurch nicht nur Wasser speichern. Vielmehr sind die SAP-Partikel bei einer wie oben beschriebenen Anordnung in der Lagenstruktur in der Lage, einen Saugfluss zu erzeugen und damit beispielsweise als Drainagematerial für die Flüssigkeitsverteilungsschicht zu dienen.

Chemisch gesehen kann es sich bei SAP um Copolymere handeln, die zum Beispiel Acrylsäure und Natriumacrylat aufweisen, wobei das Verhältnis der beiden Monomeren zu einander variieren kann. Zusätzlich werden beispielsweise Quervernetzer bei der Polymerisierung zugesetzt, die das gebildete langkettige Polymer stellenweise untereinander durch chemische Brücken verbinden. Je nach Vernetzungsgrad können die Eigenschaften des Polymers eingestellt werden. Zum Beispiel können SAP-Materialien zum Einsatz gelangen, wie sie beispielsweise aus der EP 0810 886 hervorgehen, insbesondere auch aus dem dort zitierten Stand der Technik, auf die im Rahmen der Offenbarung vollständig verwiesen wird. Eine Ausgestaltung sieht beispielsweise vor, dass SAP-Partikel eine Beschichtung aufweisen. Die Beschichtung kann sich beispielsweise in Verbindung mit einer Flüssigkeit erst auflösen, um damit eine Aufnahme der Flüssigkeit durch das SAP-Partikel überhaupt zu ermöglichen. Weiterhin kann SAP-Material eingesetzt werden, wie es aus der DE 10 2004 015 686 A1, der DE 698 217 94 und/oder der DE 10 2004 005 417 A1 jeweils hervorgeht, insbesondere in Bezug auf den Aufbau und die Struktur, die Geometrie des superabsorbierenden Polymers wie auch der dabei verwendeten Materialien und Herstellungsweisen. Auf diese Druckschriften wird im Rahman der Offenbarung beispielhaft verwiesen. Eine weitere Ausgestaltung sieht vor, dass die SAP-Partikel granulatförmig aber auch eine andere Geometrie aufweisen können, zum Beispiel faserförmig, rund oder andersförmig sein können. Fasern mit einem Gehalt an Superabsorber gehen zum Beispiel aus der DE 102 32 078 A1 wie auch aus der DE 102 51 137 A1 hervor. Auch auf diese wird im Rahmen der Offenbarung verwiesen.

Die jeweiligen Lagen können dabei gleiche oder unterschiedliche Arten an Cellulosefasern und/ oder SAP-Partikel und/oder SAP-Fasern aufweisen. Auf diese Weise kann das Aufnahmeverhalten der Einzellagen der Lagenstruktur für Flüssigkeiten definiert eingestellt werden.

Beispielsweise können in einer Lage hochpermeable SAP-Partikel und/oder SAP-Fasern eingesetzt werden, die zusammen mit SAP-Partikeln und/oder SAP-Fasern in einer weiteren Lage einen zweistufigen Absorptions- und Speichereffekt bewirken. Beispielsweise könnten in einer Lage, die der Flüssigkeitsaufnahmeschicht zugewandt ist, SAP-Partikel und/oder SAP-Fasern mit hoher Absorptionsfähigkeit und in einer weiteren Lage semipermeable SAP-Partikel und/oder SAP-Fasern vorgesehen sein. Hierdurch kann eine Pufferfunktion in der weiteren Lage erzeugt werden, die sich speziell bei mehrmaligem Aufgeben einer Flüssigkeit als vorteilhaft erweist.

In einer bevorzugten Ausführungsform der Erfindung bleiben die SAP-Partikel und/oder SAP-Fasern in der Flüssigkeitsspeicherschicht bei Flüssigkeitsaufnahme und/oder leichtem Druck beweglich zueinander angeordnet. Dieses wird ermöglicht, indem in der Flüssigkeitsspeicherschicht kein Bindemittel, beispielsweise in Form von thermoplastischen Fasern, wie Mehrkomponentenfasern, vorgesehen ist. Dadurch können sich die SAP-Partikel und/oder SAP-Fasern in der Flüssigkeitsspeicherschicht bewegen, gut ausdehnen da genügend Raum zum Quellen zur Verfügung steht, wodurch das Absorptionsvermögen der Lagenstruktur verbessert wird und gleichzeitig wieder freie Räume schaffen, in denen eine Flüssigkeit bei nochmaliger Flüssigkeitsaufgabe gespeichert werden kann. Damit kann ein derartiges Produkt insbesondere den Anforderungen an Inkontinenzprodukte gerecht werden. Zudem kann die Lage zur Verbesserung eines Dehnungsverhaltens der Lagenstruktur beitragen, zum Beispiel auch hinsichtlich einer elastischen Eigenschaft. Da die Cellulosefasern in der Flüssigkeitsspeicherschicht nicht durch ein Bindemittel miteinander verklebt beziehungsweise in Kontakt gebracht werden, sind diese Fasern und/oder das SAP-Material beweglich zueinander angeordnet. Selbst im Benutzungszustand, dem sogenannten Nasszustand tritt keine oder nur eine geringe Behinderung der Bestandteile dieser Schicht ein. Zudem wirkt das SAP-Material, insbesondere die SAP-Partikel nach dem Aufquellen, welches mit einer Quervernetzung beziehungsweise mit einer Nachvernetzung auf der Oberfläche der Partikel einhergeht, wie ein "Gleitmittel" beziehungsweise die Flüssigkeitsspeicherschicht insgesamt wie eine "Gleitschicht" zwischen der Flüssigkeitsaufnahmeschicht und der Flüssigkeitsverteilungsschicht. Vorzugsweise weisen die SAP-Partikel im trockenen Zustand an ihrer Oberfläche bereits überwiegend bogenförmige Abschnitte auf, so dass im gequollenen Zustand bevorzugt kugelförmige Partikel entstehen, wodurch die äußere Gestalt der Partikel beim Übergang vom trockenen Zustand in den Nasszustand nur unwesentliche Änderungen erfährt.

Durch die überwiegend kugelförmigen Partikel wird bei Benutzung der absorbierenden Struktur eine Beweglichkeit innerhalb der Flüssigkeitsspeicherschicht und zwischen dieser und den einzelnen Schichten der Lagenstruktur erreicht, die sich in einem verbesserten Tragekomfort eines daraus gefertigten Wegwerfprodukts widerspiegelt. Die Beweglichkeit der SAP-Partikel oder SAP-Fasern in der Flüssigkeitsspeicherschicht ist so zu verstehen, dass die Partikel im befeuchteten und gequollenen Zustand, das heißt im Nasszustand, bei Belastung noch in der Lage sind, sich zu bewegen und aneinander abzugleiten. Im entlasteten Zustand kann es sogar zu einer partiellen Rollbewegungen kommen, die dafür sorgt, dass eine während des Gebrauchs zusammengedrückte innere Lage in Form einer Flüssigkeitsspeicherschicht nach ihrer Entlastung weitestgehend wieder in den Originalzustand zurückkehren kann. Im nassen Zustand entkoppeln sich die Außen-Lagen der Lagenstruktur und der zu festem Gel vergrößerte SAP bildet eine bewegliche, gleitende Zwischenlage aus, bei dem das Gel wie ein Kugel-Lager die seitliche/x-y-flächige Verschiebung gegeneinander einfach zulässt. Dies sorgt für bestehenden Tragekomfort, ohne einen Verlust der Materialintegrität. Die durch einen nassfesten Dispersionsbinder gebundenen Außenlagen behalten ihre gewünschten textilen Eigenschaften mit Rückstellkräften bei, so dass das Material im Ganzen die Fähigkeit beibehält, einer vorgegebenen Form zu folgen. Auch bei Veränderung der Form verhält es sich nicht plastisch wie herkömmliche nasse Lagen aus Fluff-Pulp und SAP. Die Beweglichkeit der Lagenstruktur sowie eine textile Eigenschaft in Oberflächennähe werden somit beibehalten.

So werden beispielsweise einwirkende Druck-, Scher- oder Zugkräfte, die durch die Benutzung eines Absorberproduktes von außen auf die Lagenstruktur aufgebracht werden können, durch die Beweglichkeit der Flüssigkeitsaufnahme-, Flüssigkeitsspeicher- und Flüssigkeitsverteilungsschicht zueinander kompensiert, so dass eine Dauerverformung oder gar eine Delamination der Schichten im Gebrauch der Absorberstruktur vermieden wird.

Dabei ist es nicht ausgeschlossen, dass vereinzelte Bestandteile des Bindemittels der Flüssigkeitsaufnahmeschicht und/oder der Flüssigkeitsverteilungsschicht in die Flüssigkeitsspeicherschicht hineinragen können und sogar die Cellulosefasern oder vereinzelte SAP-Partikel oder -fasern ganz oder teilweise ummanteln. Hierdurch entsteht eine erhöhte Verbundfestigkeit, wobei insbesondere die äußeren Oberflächen der Flüssigkeitsspeicherschicht im Grenzbereich zur jeweiligen Flüssigkeitsaufnahme- beziehungsweise Flüssigkeitsverteilungsschicht stabilisiert werden, jedoch bleibt die zum Erhalt eines guten Tragekomforts erforderlich Beweglichkeit der Schichten zueinander und der Flüssigkeitsspeicherschicht selbst erhalten.

So sieht beispielsweise eine weitere Ausführungsform für eine absorbierende Struktur sieht vor, dass zumindest ein Teil des Bindemittels, vorzugsweise der Bikomponentenfasern der Flüssigkeitsverteilungsschicht und/oder Flüssigkeitsaufnahmeschicht in einem jeweils dazwischen liegenden Grenzbereich zur Flüssigkeitsspeicherschicht mit dem Cellulosematerial der Flüssigkeitsspeicherschicht vermischt sind.

Das Bindemittel weist in einer bevorzugten Ausbildung der Erfindung thermoplastische Fasern in Form von Bikomponentenfasern auf. Beispielsweise können Bico-Fasern, insbesondere Kern-Mantel-Fasern eingesetzt werden, bei denen der Mantel einen geringeren Schmelzpunkt aufweist als der Kern. Es ist ebenfalls vorgesehen, dass die Bikomponentenfasern zumindest ein PET umfassen. Vorteilhafterweise umfassen die Bikomponentenfasern zumindest ein Polyethylen, vorzugsweise ein LDPE oder ein LLDPE. In einer Bikomponentenfaser mit einer Kern-Mantel-Struktur ist ein PET oder ein Polypropylen enthaltendes Polymer im Kern und ein Polyethylen enthaltendes Polymer im Mantel vorgesehen. Durch Erwärmung werden die Bikomponentenfasern zumindest soweit aufgeweicht, dass sie eine klebrige Oberfläche bilden, an der Cellulosefasern wie auch andere Komponenten der Schicht aber auch Komponenten benachbarter Schichten festgefügt werden bei Abkühlung. Gemäß einer Ausgestaltung können cellulosebindende Fasern eingesetzt werden, wie sie aus der DE 69808061 hervorgehen, auf die im Rahmen der Offenbarung verwiesen wird.

Darüber hinaus können die Lagen der Flüssigkeitsverteilungsschicht und/oder der Flüssigkeitsspeicherschicht und/oder der Flüssigkeitsaufnahmeschicht innerhalb des jeweiligen Grenzbereiches zumindest teilweise ineinander übergehen.

Dadurch wird gewährleistet, dass die Schichten des Lagenverbunds der absorbierenden Struktur im Vergleich zu herkömmlichen Produkten nicht nur im trockenen Zustand, sondern auch im Nasszustand einen sehr guten Zusammenhalt aufweisen und trotzdem zueinander beweglich sind, wodurch eine hinreichende Nassreißfestigkeit unter Beibehaltung eines guten Tragekomforts erreicht wird. Die Reißfestigkeit der erfindungsgemäßen Lagenstruktur liegt im trockenen Zustand im Bereich zwischen 15 - 27 N und im Nasszustand zwischen 4 - 7 N, wobei die Biegesteifigkeit der Lagenstruktur, die für den Tragekomfort maßgeblich ist, im nassen Zustand nur noch etwa 3 - 8 % der Steifigkeit des trockenen Zustands aufweist.

Eine weitere Ausführungsform der Erfindung sieht vor, dass die Flüssigkeitsaufnahmeschicht und/oder die Flüssigkeitsverteilungsschicht stärker verdichtet sind, als die Flüssigkeitsspeicherschicht. Die absorbierende Struktur kann derart ausgebildet sein, dass die Flüssigkeitsverteilungsschicht eine erste und eine zweite Oberfläche aufweist, wobei die erste Oberfläche in Kontakt mit der Flüssigkeitsspeicherschicht steht und wobei die Flüssigkeitsverteilungsschicht an ihrer zweiten Oberfläche stärker verdichtet ist, als an ihrer ersten Oberfläche.

Die Flüssigkeitsspeicherschicht ist vorzugsweise als voluminöses Vlies ausgeführt und umfasst zum überwiegenden Teil Cellulosefasern. Diese können ebenso wie in der Flüssigkeitsaufnahmeschicht oder Flüssigkeitsverteilungsschicht behandelt oder unbehandelt sein.

Obwohl die einzelnen Schichten des Lagenmaterials zum überwiegenden Teil Cellulosefasern und vereinzelt Bindefasern, vorzugsweise thermoplastische Bindefasern aufweisen, ist es nicht ausgeschlossen, dass auch weitere Fasern natürlicher oder synthetischer Art, beispielsweise thermoplastische Fasern, vorzugsweise Spunbondfasern, Meltblownfasern, Stapelfasern und ähnliches in den Schichten angeordnet sind. Durch die Wahl der Anordnung und/oder Präparation dieser Fasern kann ebenfalls ein Eigenschaftsgradient innerhalb einer Schicht des Lagenmaterials oder über ein oder mehrere Schichten hinweg erzeugt werden. Zur Erzeugung dieser synthetischen Fasern werden Polymere bevorzugt, die zum überwiegenden Teil Polypropylen, Polyethylen, Polyester, Polyamid aufweisen.

Durch Verwendung thermoplastischer Fasern, beispielsweise in der Flüssigkeitsaufnahmeschicht kann ein erneutes Benetzen mit Flüssigkeit und Weiterleiten dieser in die Flüssigkeitsspeicherlage verbessert werden. Hierzu können angepasst an einen Einsatzzweck die Fasern der Flüssigkeitsaufnahmeschicht entsprechend ausgerüstet sein, zum Beispiel durch hydrophobe Ausrüstung, wodurch eintreffende Flüssigkeit sofort in Richtung hydrophiler Fasern fließt oder hydrophile Ausrüstung, wodurch gezielt Kapillarwege geschaffen werden, die einen schnelleren Flüssigkeitstransport ermöglichen.

In einer Weiterbildung der Erfindung umfasst die Flüssigkeitsaufnahmeschicht, die Flüssigkeitsspeicherschicht und/oder die Flüssigkeitsverteilungsschicht Airlaid-Lagen oder besteht aus diesen.

Zudem kann die absorbierende Struktur eine Trägerschicht, vorzugsweise eine Tissue-Schicht aufweisen. Dabei kann die Trägerschicht an einer Außenseite der Flüssigkeitsverteilungsschicht angeordnet sein.

Außerdem kann die absorbierende Schicht eine weitere Binderschicht, beispielsweise eine Latexschicht aufweisen, die auf der Flüssigkeitsverteilungsschicht und/oder der Flüssigkeitsaufnahmeschicht angeordnet ist.

Demgemäß weist die Lagenstruktur eine erste und eine zweite Oberfläche auf, wobei die erste und/oder die zweite Oberflache eine Latex-basierte Bindemittelschicht aufweist. In der äußeren Flüssigkeitsaufnahmeschicht der absorbierenden Struktur wird bevorzugt ein unbehandelter Cellulose-Pulp-Typ eingesetzt, um mit längeren Fasern mehr Volumen zu generieren.

Die erste und/oder die zweite Oberfläche einer Lagenstruktur kann beispielsweise eine äußere Oberfläche einer Flüssigkeitsaufnahmeschicht oder einer Flüssigkeitsverteilungsschicht oder einer Trägerschicht, vorzugsweise einer Tissue-Schicht sein. Die absorbierende Struktur kann einen Gradienten hinsichtlich einer Porenstruktur aufweisen, die ein Abfließen von der Flüssigkeitsaufnahmeschicht hin zur Flüssigkeitsverteilungsschicht unterstützt. Die Gradientenstruktur kann sich innerhalb einer Schicht wie aber auch Ober mehrere Schichten erstrecken. Der Gradient kann bevorzugt ein Ansteigen der Kapillarkraft bewirken. Ein Gradient ist zum Beispiel durch die Form der Ablage der Cellulosefasern einstellbar, durch zusätzliche Verdichtung und/oder durch Verringerung der Poren mittels zusätzlicher Mittel, zum Beispiel dem Zuführen von Flüssigkeit oder Binder, welches die Poren verkleinert oder aber teilweise verstopft. Beispielsweise ist dieses mittels einer Latexbenetzung möglich.

Die Erfindung stellt ein Verfahren zur Herstellung einer absorbierenden Struktur zur Verfügung, welches mindestens die folgenden Schritte umfasst:

- Ablegen von Fluffpulp, SAP und Bindefasern als zweite Lage auf einer Tragschicht,
- Ablegen von Fluffpulp und SAP auf der zweiten Lage als dritte Lage,
- Ablegen einer vierten Lage aus Fluffpulp und Bindefasern auf der dritten Lage,
- Aufbringen eine Latex-basierten Bindemittelschicht auf mindestens eine Au-ßenoberfläche der Lagen,
- Zuführen der Lagen zu einem Kalander, der einen Kalanderspalt hat,
- Verdichtung der Lagen im Kalanderspalt.

Die dritte Lage funktioniert gemäß einer Ausgestaltung als Aufnahmelage, die zweite Lage als Flüssigkeitsspeicherlage und die zweite Lage als Flüssigkeitsverteilungslage. Bevorzugt bestehen daher die jeweiligen Lagen aus den angeführten jeweiligen Komponenten, ohne dass eine weitere Komponente in den jeweiligen Lagen zusätzlich vorhanden ist oder zugeführt wird. Eine entsprechende Vorrichtung weist gemäß einer Ausgestaltung daher auch nur eine Zuführung für diese jeweiligen Komponenten auf oder ist gemäß einer weiteren Ausgestaltung so ausgelegt, dass nur diese Komponenten in der jeweiligen Lage zugeführt werden und andere Komponenten in der jeweiligen Lage, die alternativ oder ergänzend zuführbar sind, abgesperrt werden können und auch abgesperrt sind.

Eine Weiterbildung der Erfindung sieht ein Verfahren zur Herstellung einer absorbierenden Struktur vor, welches mindestens die folgenden Schritte umfasst:

- Ablegen von zumindest einer ersten Lage, bevorzugt einer Airlaid-Lage, die vorzugsweise zumindest ein Cellulosematerial, ein Bindemittel, vorzugsweise Multikomponentenfasern umfasst, zur Ausbildung einer Flüssigkeitsaufnahmeschicht,

- Ablegen einer Lage, vorzugsweise einer Airlaid-Lage zur Ausbildung einer Flüsigkeitsspeicherschicht, die zumindest ein Cellulosematerial, vorzugsweise Cellulosefasern und ein superabsorbierendes Polymer, vorzugsweise SAP-Partikel und/oder SAP-Fasern und kein Bindemittel umfasst,

- Ablegen einer dritten Lage, vorzugsweise einer Airlaid-Lage zur Ausbildung einer Flüssigkeitsverteilungsschicht, die zumindest ein Cellulosematerial und ein Bindemittel, vorzugsweise Multikomponentenfasern umfasst,

- Aufbringen einer Latex-basierten Bindemittelschicht auf eine Außenoberfläche, vorzugsweise auf die so erhaltene Lagenstruktur,

- vorzugsweises Durchführen der Lagenstruktur durch ein Heizmittel, um die Lagenstruktur zu binden,

- Zuführen zumindest einer Lage, bevorzugt der Lagenstruktur zu einem Kalander, umfassend mindestens eine Glattwalze und eine Gegenwalze, die einen Kalanderspalt bilden,

- lockere Verdichtung der zumindest einen Lage, bevorzugt der Lagenstruktur im Kalanderspalt.

Die Erfindung sieht weiterhin eine Vorrichtung zur Herstellung der obigen absorbierenden Struktur vor, aufweisend zumindest:

- ein Siebband zur Ablage von Lagen zur Ausbildung einer Lagenstruktur,

- eine erste Formungseinrichtung, über welche zumindest Fluffpulp, SAP und Bindefasern auf dem Siebband aufbringbar sind, um eine Lage zu bilden,

- eine zweite Formungseinrichtung, über die Fluffpulp und SAP auf dem Siebband aufbringbar sind, um eine weitere Lage zu bilden,

- eine dritte Formungseinrichtung, über die Bindefasern und Fluffpulp als weitere Lage auf dem Siebband aufbringbar sind,

- zumindest eine Auftragsstation, über die eine Latex-basierte Bindemittelschicht auf eine äußere Oberfläche der Lagenstruktur aufbringbar ist,

- eine Verdichtungsstation, vorzugsweise ein Kalander, über welche die Lagenstruktur verdichtbar ist.

Die zweite Formungseinrichtung ist so ausgestaltet, dass mittels ihr kein Bindemittel zugeführt wird beziehungsweise zugeführt werden kann. Beispielsweise kann es dazu an notwendigen Anschlüssen fehlen, an einer Bindemittelauftragskomponente oder einer Bindemittelzuführungskomponente. Auch kann eine Verbindung zu einem Bindemittelspeicher unterbrochen sein.

Gemäß einem weiteren Gedanken der Erfindung wird eine Vorrichtung zur Herstellung einer absorbierenden Struktur vorgeschlagen, die zumindest die folgenden Komponenten aufweist:

- ein Siebband zur Ablage von Lagen, vorzugsweise Airlaid-Lagen zur Ausbildung einer Lagenstruktur,

- eine erste Formungseinrichtung, vorzugsweise eine Airlaid-Formungseinrichtung, über welche zumindest ein Cellulosematerial, vorzugsweise Cellulosefasern abziehbar sind,

- eine zweite Formungseinrichtung, über die ein Bindemittel, vorzugsweise Mehrkomponentenfasern abziehbar sind, um gemeinsam mit den Cellulosefasern eine erste Lage zu bilden

- eine dritte Formungseinrichtung, über die ein superabsorbierendes Polymer SAP, vorzugsweise SAP-Partikel und/oder SAP-Fasern auf der ersten Lage ablegbar sind,

- eine vierte Formungseinrichtung, über welche zumindest Cellulosefasern und ein superabsorbierendes Polymer SAP, vorzugsweise SAP-Partikel und/oder SAP-Fasern auf der ersten Lage ablegbar sind und vorzugsweise eine zweite Lage bilden,

- eine Ablagevorrichtung für eine dritte Lage, umfassend ein Cellulosematerial und ein Bindemittel, vorzugsweise Mehrkomponentenfasern;

- vorzugsweise ein Auftragsmittel, über das ein Bindemittel, vorzugsweise eine Latexschicht, aufbringbar ist,

- ein Heizmittel, in welchem Bikomponentenfasern und/oder das Bindemittel aktivierbar sind,

- eine Walzenanordnung, vorzugsweise ein Kalander, über welche die Lagenstruktur locker verdichtbar ist.

Eine Dosierung der SAP-Partikel und/oder SAP-Fasern kann sich über eine Breite des Materials unterscheiden. Auch besteht die Möglichkeit, unterschiedliche SAP-Materialen über die Breite des Materials an verschiedenen Orten wie auch an gleichen Orten anzuordnen, insbesondere abzulegen. Eine Ausgestaltung sieht vor, dass über eine Dicke des Materials SAP-Partikel in einer Schicht unterschiedlich angeordnet werden. Eine Positionskontrolle erfolgt beispielsweise über eine gezielte Ausrichtung der SAP-Zuführung. Auch besteht die Möglichkeit, diese Positionskontrolle automatisch auszuführen, zum Beispiel über Sensoren, bildanalysierende Verarbeitung oder ähnliches. Auch besteht die Möglichkeit, dass die Lage der SAP-Partikel und/oder SAP-Fasern in der Schicht automatisch geprüft wird, zum Beispiel mittels Detektierung der SAP-Partikel und/oder SAP-Fasern. Dazu können SAP-Partikel und/oder SAP-Fasern beispielsweise eine detektierbare Kennzeichnung, zum Beispiel ein spezielles Material, ein Farbe oder anderes aufweisen. Dieses erlaubt beispielsweise eine Korrektur während des laufenden Herstellungsverfahrens.

Die Weiterbearbeitung der absorbierenden Strukturen kann direkt im Anschluss nach der Lagenerstellung erfolgen. Die absorbierenden Strukturen können aber noch zusammenhängend auch aufgerollt oder über eine Festooning-Einheit transportierbar gemacht werden. Eine Weiterverarbeitung kann sodann an einem anderen Ort erfolgen. Eine Weiterverarbeitung kann zum Beispiel eine Beschichtung, ein weiteres Laminieren mit ein oder mehreren anderen Schichten, ein Schneiden in Längs- und/oder Querrichtung, ein weiteres Verdichten und/oder Bondieren, ein Recken und/oder einen sonstigen Schritt umfassen.

Komponenten einer Airlaid-Herstellungsvorrichtung und deren jeweiliger Einsatz gehen zum Beispiel aus der DE 10 2004 009 556 A1 betreffend die Herstellung einer Faserbahn aus Cellulosefasern, der DE 10 2004 024 551 B4 betreffend eines Formkopfes wie auch eines Verfahrens zur Herstellung einer Airlaid-Schicht, aus der DE 10 2004 056 154 A1 betreffend eine Transportvorrichtung hervor. Weiterhin geht aus der DE 103 270 26 A1 ein Verfahren zur Herstellung eines Faservlieses nach einem Airlaid-Verfahren wie auch eine dafür geeignete Faser hervor. Aus der DE 199 183 43 A1 geht wiederum ein Airlaid-Verfahren und eine Airlaidlage hervor, bei der auch eine Bindefaser zum Einsatz gelangt. Aus der WO 2005/080655 A1 ist wiederum der Aufbau einer Airlaidanlage mit verschiednen weiteren Komponenten und deren Lageanordnung und Zweck entnehmbar. Eine Detektierung von SAP und deren kontrollierte Abgabe und eventuelle Korrektur ebenso wie die Erstellung voneinander getrennter absorbierender Strukturen geht zum Beispiel aus der WO03/034963 A2 hervor.

Im übrigen wird im Rahmen der Offenbarung der Erfindung auch auf die beiden jeweils noch nicht veröffentlichten Prioritätsanmeldungen US 12/657,987 mit dem Titel "Flexible, Highly Absorbent Material", Erfinder Röttger et.al. und der DE 10 2010 006 228 mit dem Titel" Flexibles, stark absorbierendes Material" und deren jeweiliger vollständiger Inhalt verwiesen. Die dort hervorgehenden jeweiligen Merkmale sind vollumfänglich auch Bestandteil dieser Beschreibung.

Die oben genannten wie auch die dort im Stand der Technik als Entgegenhaltung genannten Druckschriften geben weitere Möglichkeiten an, wie die Vorrichtung ausgestaltet sein kann. Im Rahmen der Offenbarung der Erfindung wird auf diese Druckschriften wie auch auf den in der Einführung genannten Stand der Technik vollumfänglich verwiesen.

Die absorbierende Struktur kann in einem Wegwerfprodukt, beispielsweise für den Hygienebereich eingesetzt werden. Auch kann die absorbierende Struktur der Wegwerfartikel selbst sein. Vorzugsweise werden diese Wegwerfprodukte für Babywindeln, Damenhygiene, Inkontinenzprodukten eingesetzt.

Darüber hinaus können derartige Wegwerfprodukte m Bereich von

- Medikalprodukten, wie beispielsweise Saugmaterialien und Unterlagen, oder
- industriellen Produkten, wie beispielsweise absorbierende Materialen zur Aufnahme von Flüssigkeiten genutzt werden.

Dazu kann die absorbierende Struktur selbst zumindest eine Außenfläche, bevorzugt beide Außenflächen eines Produkts bilden. Die absorbierende Struktur kann aber auch zumindest an einer Seite, beispielsweise auch an jeder Seite mit einer zusätzlichen Lage zumindest abgedeckt, bevorzugt verbunden sein.

Weitere vorteilhafte Ausgestaltungen und Weiterbildungen der vorliegenden Erfindung werden anhand der nachfolgenden Beispiele, die auch in den Zeichnungen dargestellt sind, näher erläutert. Die dabei dargelegten Merkmale sind nicht auf die einzelne Ausgestaltung beschränkt sondern können untereinander wie auch mit den weiter oben beschriebenen Merkmalen zu zusätzlichen Weiterbildungen verknüpft werden, die im einzelnen jedoch hier nicht ausgeführt werden.

Es zeigen:

Fig. 1 und Fig. 2:    schematische Ansichten von Querschnitten verschiedener bondierter absorbierender Strukturen,

Fig. 3    eine schematische Ansicht einer Ausgestaltung einer Herstellungsvorrichtung,

Fig. 4    in schematischer Ansicht ein weiteres bevorzugtes Ausführungsbeispiel einer absorbierenden Struktur,

Fig. 5    schematisch eine Probe A mit einer Probenbezeichnung VH 460.103 in einem Schnitt und in einer Seitenansicht in Längsrichtung,

Fig. 6    einen schematischen Schnitt durch eine Probe B mit einer Bezeichnung VH 600.101 im Querschnitt und in einer Seitenansicht

Fig. 7    eine prinzipielle Darstellung einer Einspannung einer Probe zwischen Spannbacken einer Messapparatur für Zugversuche,

Fig. 8    die Ergebnisse der Zugversuche zur Bestimmung einer Scherfestigkeit, wiedergegeben als Tabelle 5, und

Fig. 9    eine schematische Ansicht einer weiteren Ausgestaltung einer Herstellungsvorrichtung .

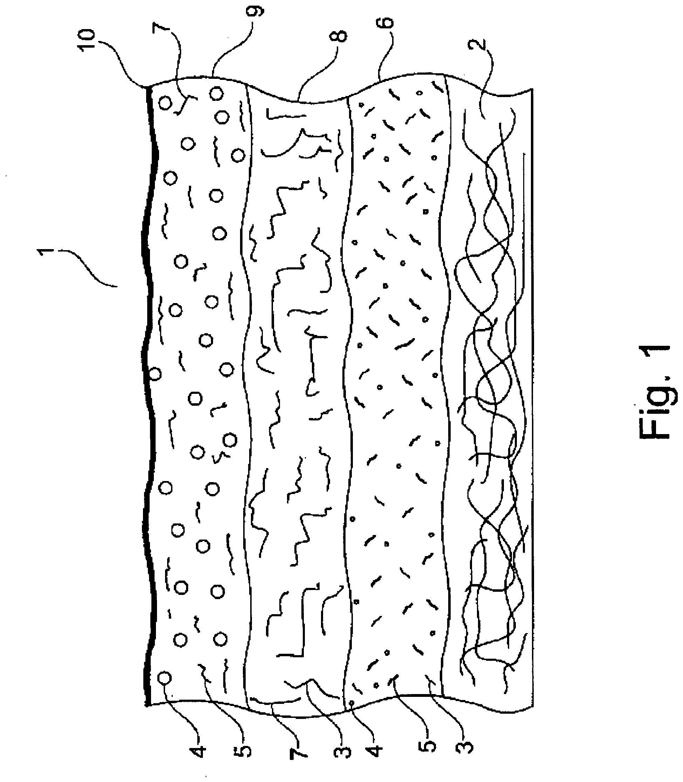

Die in Fig. 1 dargestellt absorbierende Lagen-Struktur 1 weist folgende Schichten auf:

- Eine Tragschicht 2, die ein Tissue beinhaltet und die erste Lage der Lagen-Struktur bildet,
- eine zweite Lage 6 aus Cellulose-Fluff-Pulp 3, SAP-Partikeln 4 und Bindefasern 5, beispielsweise Bikomponentenfasern,
- eine dritte Lage 8 aus Cellulose-Pulp 7 und Cellulose-Fluff-Pulp 3,
- eine vierte Lage 9 aus Cellulose-Pulp 7, bevorzugt SAP-Partikeln 4, die aber auch weggelassen sein können, und Bindefasern 5 sowie
- eine Lage aus einem Bindemittel 10, beispielsweise einem Latex-Binder.

Die Lagen der Lagen-Struktur sind übereinander angeordnet und in einem anschließenden Kalandrierprozess unter Anwendung von Wärme und Druck miteinander verbunden worden sind. Dabei sind die äußeren Lagen der Struktur stärker verdichtet, als die mittleren Lagen. Das Lagenmaterial 1 kann auch ohne die Tragschicht 2 ausgebildet sein. Die zweite Lage 6 übernimmt allein oder in Kombination mit der Tragschicht 2 die Funktion einer Flüssigkeitsaufnahmeschicht in der Lagen-Struktur. Aufgrund der in der Lage 6 vorhandenen SAP-Partikel 4 hat diese Lage auch eine Speicherfunktion, die durch die Lage 8 verstärkt wird. Die Lage 8 weist Fluff-Pulp 3 und Cellulose-Pulp 7 auf. Sie ist nur leicht verdichtet und weist ein hohes Lückenvolumen auf. Die Lage 9 umfasst Cellulose-Pulp 7, Bindefasern 5 und SAP-Partikel 4 und kann als Flüssigkeitsverteilungsschicht wirken. Auf der Lage 9 ist eine äußere Lage aus einem Bindemittel 10, beispielsweise einem Latex-Binder angeordnet.

In Abhängigkeit von der Wahl des Ablege- und Bindeprozesses, der in diesem Falle ein in-line-Prozess ist, können die einzelnen Lagen eine unterschiedliche Verdichtung aufweisen, die vorteilhafterweise an den beiden äußeren Oberflächen der Lagenstruktur höher ist als in ihrem inneren Bereich. So wie anhand der Fig. 1 dargelegt, kann eine Funktionalität der einzelnen Lagen auch andersherum sein: so ist als Aufnahmelage für Flüssigkeit die vierte Lage vorgesehen, wobei sodann kein SAP in der vierten Lage vorgesehen ist, wohingegen die Tragschicht in Form eines Tissues eine Sperrschicht darstellt, da sie am engporigsten von allen Lagen und damit den größten Widerstand hinsichtlich eines Flüssigkeitsdurchgangs in diesem Fall liefert.

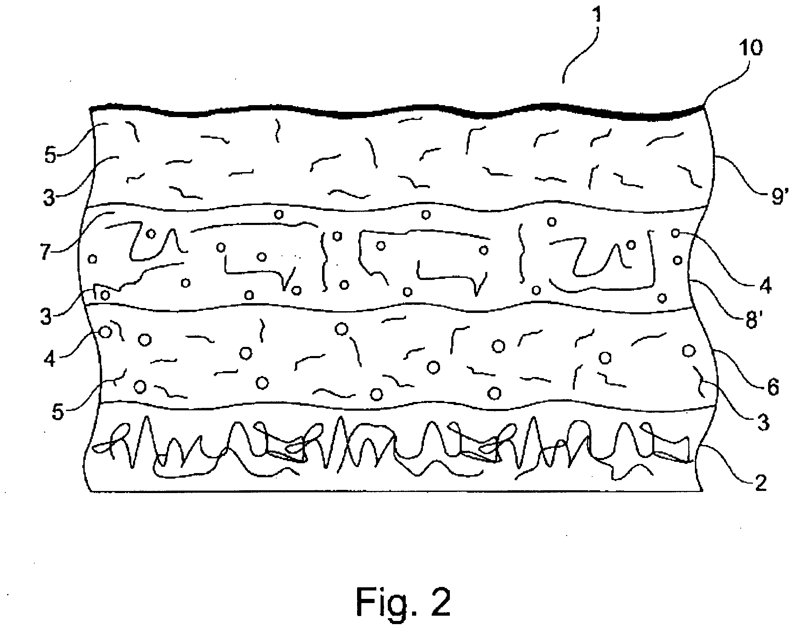

Die in Fig. 2 dargestellt absorbierende Lagen-Struktur 1 weist folgende Schichten auf:

- Eine Tragschicht 2, die ein Tissue beinhaltet und die erste Lage der Lagen-Struktur bildet,
- eine zweite Lage 6 aus Cellulose-Fluff-Pulp 3, SAP-Partikeln 4 und Bindefasern 5, beispielsweise Bikomponentenfasern,
- eine dritte Lage 8' aus Cellulose-Pulp 7, Cellulose-Fluff-Pulp 3 und SAP-Partikeln 4,
- eine vierte Lage 9' aus Cellulose-Pulp 7 und Bindefasern 5 sowie
- eine Außenlage aus einem Latex-Binder 10.

Die Lage 8' weist auf Grund ihrer Zusammensetzung, vorzugsweise den Cellulose-Fluff-Pulp, eine sehr geringe Verdichtung im Vergleich zu den angrenzenden Lagen auf. Hierdurch haben die in der Lage 8' vorgesehenen SAP-Partikel eine hohe Beweglichkeit im Falle einer Belastung durch Flüssigkeitsaufgabe oder Scherbeanspruchung, beispielsweise durch den Benutzer. Diese Beweglichkeit innerhalb der Lage 8' ermöglicht eine Entkopplung der an ihr angrenzenden Lagen, so das hierdurch die Lagen der Struktur relativ zueinander beweglich sind. Die im Gebrauchszustand aufgequollenen SAP-Partikel können auf Grund der geringen Verdichtung der Lage 8' aneinander abgleiten und bilden somit die Voraussetzung für die in der Lage 8' beobachtete Gleitwirkung, die bei einer Belastung der Lagen-Struktur ein Zerreißen oder eine Delamination verhindern.

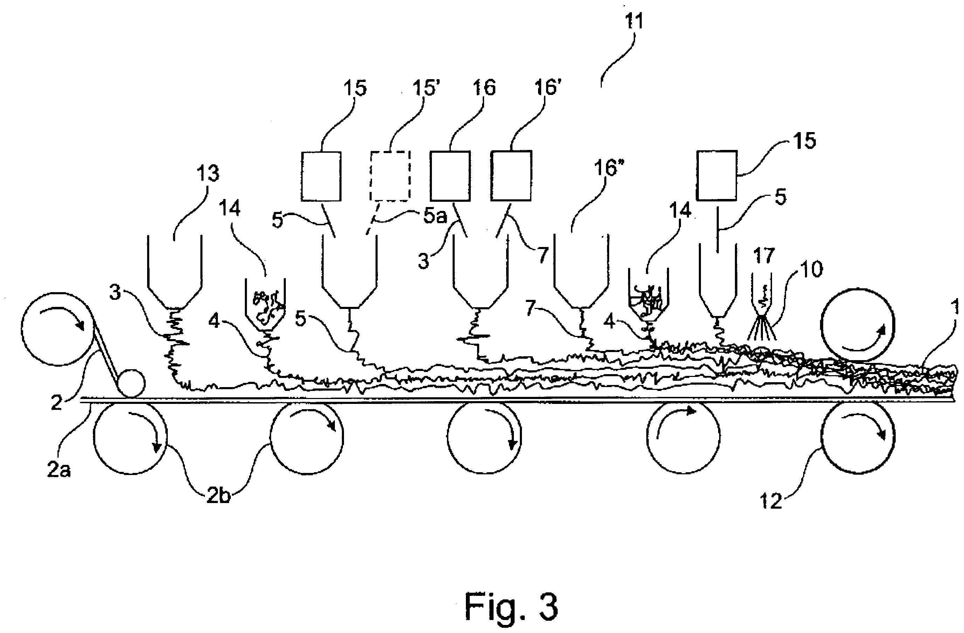

Fig. 3 zeigt eine schematische Darstellung einer möglichen Ausgestaltung für eine Vorrichtung 11 zur Herstellung einer absorbierenden Lagen-Struktur 1. Dargestellt ist ein in-line-Prozeß mit einer Abrollvorrichtung zur Bereitstellung eines Airlaid-Materials 2, welches auf einem Siebband 2a mit Antriebswalzen 2b abgelegt ist, einer ersten Formungseinrichtung 13 zur Bereitstellung von Fluff-Pulp 3, einer Formungseinrichtung 14 zur Bereitstellung eines Absorbers, beispielsweise SAP-Partikel 4, eine weitere Formungsvorrichtung 15, 15' zur Bereitstellung von Bindefasern 5 und gegebenenfalls weiteren Bindefasern 5a, um eine erste Lage 6 zu bilden, die auf der Tragschicht 2 abgelegt wird. Über Formungseinrichtungen 16 und 16' werden Fluff-Pulp 3 und Pulp 7 bereitgestellt, um eine dritte Lage 8 zu bilden, die auf der zweiten Lage 6 abgelegt wird. Eine Formungsvorrichtung 16" zur Bereitstellung von Pulp 7, sowie Formungsvorrichtungen 14 beziehungsweise 15 zur Bereitstellung von SAP-Partikeln 4 beziehungsweise Bindefasern 5 dienen der Ausbildung einer vierten Lage 9, die auf der dritten Lage abgelegt wird. Über eine Einrichtung 17 wird ein Bindemittel 10, beispielsweise ein Latex-Binder auf die Lage 9 aufgebracht, beispielsweise durch Aufsprühen oder Rakeln. Die Lagen-Struktur kann nach dem Bindemittelauftrag mittels Wärme aktiviert werden (nicht dargestellt) und durchläuft anschließend eine Einrichtung 12 zum Verdichten der Lagen-Struktur. Die Einrichtung 12 ist beispielsweise ein Kalander mit einer Anordnung von Glattwalzen. Es kann aber auch eine Infrarot-Heizung, ein Ofenabschnitt oder eine sonstige Beheizung zur Aktivierung der Bindefasern vorgesehen sein, die bindemittelhaltigen Schichten untereinander aber auch jeweils miteinander sich verbinden.

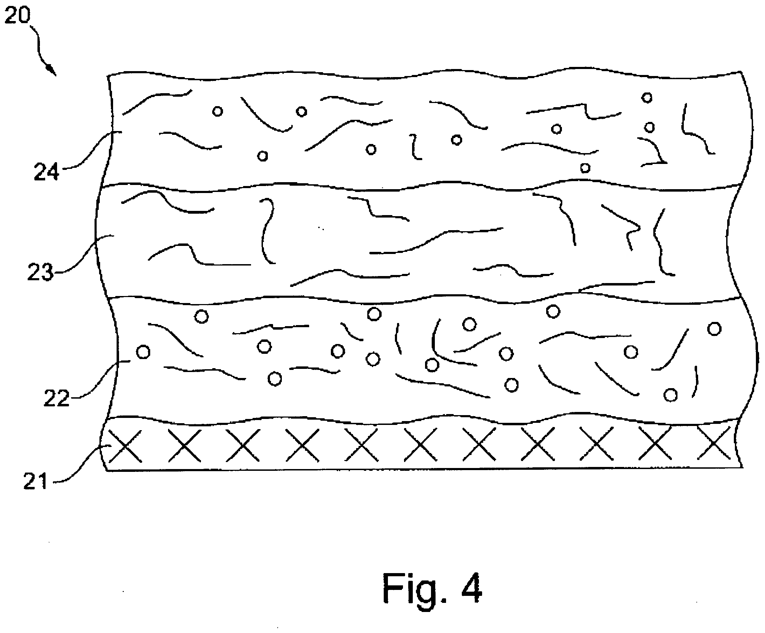

Fig. 4 zeigt in schematischer Ansicht ein weiteres bevorzugtes Ausführungsbeispiel einer absorbierenden Struktur 20. Auf einem Paper-Tissue 21 als eine erste Lage ist eine zweite Lage 22 angeordnet. Die zweite Lage 22 weist Pulp, bevorzugt behandelten Pulp, SAP und ein Bindemittel, bevorzugt Klebefasern zum Beispiel in Form von Bico-Fasern auf. Das SAP kann beispielsweise als Partikel und/oder auch als Faser eingebracht werden. Die zweite Lage 22 dient als Back-up-Speicher und kann auch zur Verteilung von Flüssigkeit genutzt werden, die durch einen darüber angeordneten Flüssigkeitsspeicher hindurchgelangt. Die zweite Lage 22 kann beispielsweise über die Verteilung des SAP, durch die Einstellung der Porengröße und/oder anderer Mittel eine derartige Funktion ermöglichen. Das Paper-Tissue 21 ist bevorzugt soweit verdichtet, dass es als Sperre gegenüber in der zweiten Lage enthaltenen und durchströmenden Flüssigkeit wirkt. Auf der zweiten Lage 22 ist eine weitere, dritte Lage 23 angeordnet, die aus Pulp, vorzugsweise aus unbehandeltem Pulp und/oder behandeltem Pulp, und SAP besteht. Es ist dort kein Bindemittel enthalten. Die dritte Lage 23 ist eine Flüssigkeitsspeicherlage. Auf der dritten Lage 23 befindet sich eine vierte Lage 24. Diese weist Pulp, bevorzugt unbehandelten Pulp, und ein Bindemittel, bevorzugt Klebefasern, zum Beispiel Bico-Fasern auf. Bevorzugt besteht die vierte Lage 24 aus diesen Materialien. Des weiteren dient die vierte Lage 24 als Aufnahmelage, d. h. tritt als erstes mit einer auftreffenden Flüssigkeit in Kontakt und leitet diese an die nachfolgenden Lagen weiter. Die Erste Lage wie auch die vierte Lage 24 werden des Weiteren bevorzugt mit jeweils einem zusätzlichen Bindemittel beaufschlagt, zum Beispiel durch Imprägnierung, Druckauftrag, im Sprayverfahren oder auf andere Weise. Bevorzugt wird ein Latexauftrag jeweils aufgebracht. Das Bindemittel, das auf die erste und die vierte Lage jeweils von außen aufgebracht wird, ist vorzugsweise das gleiche Bindemittel, insbesondere der gleiche Latexauftrag. Eine Weiterbildung sieht vor, dass das auf die jeweilige Außenseite aufgebrachte Bindemittel sich voneinander unterscheidet. Das Bindemittel wird bevorzugt gemäß einer Ausgestaltung so dosiert aufgebracht, dass es nur in die erste beziehungsweise vierte Lage eindringt. Eine weitere Ausgestaltung sieht vor, dass es zumindest im Wesentlichen nur an der Oberfläche der ersten beziehungsweise vierten Lage verbleibt. Eine wiederum andere Ausgestaltung sieht vor, dass es von der ersten Lage 21 in die dazu benachbarte zweite Lage 22 eindringt, hingegen nicht in die dritte Lage 23.

**Beispiele**

In den folgenden Beispielen werden die Lagenstruktur sowie deren Herstellung gemäß der vorliegenden Erfindung näher erläutert. Wie nachstehend beschrieben, wurden erfindungsgemäße Proben und Vergleichsproben hergestellt und anschließend an diesen Messungen zur Ermittlung der Dicke, der Beugelänge, der Biegsteifigkeit, der Reißfestigkeit, der Bindestärke, der Flüssigkeitsaufnahmekapazität sowie der Scherfestigkeit durchgeführt.

Es wurden zwei Materialien A und B unter Verwendung von Airlaid-Schichten entsprechend des Vorschlags einer neuen absorbierenden Struktur hergestellt. Beide Materialien umfassen drei Schichten, die auf einer Trägerschicht aus einem Tissue abgelegt worden sind. Nach der Formung dieser Lagenstruktur erfolgte eine Besprühung der beiden äußeren Oberflächen der Lagenstruktur mittels eines Dispersionsbinders aus einer Wasser-basierten EVA-Latex-Mischung.

Das Material A (VH460.103) weist nach einem Kalandrierschritt ein Flächengewicht von 460 g/m$^2$ bei einer

Dicke von 5 mm (bei 0,5 kPa) und einer Dichte von 0,092 g/cm$^3$ auf, während das Material B (VH600.101) bei analoger Herstellung ein Flächengewicht von 600 g/m$^2$ bei einer Dicke von 6 mm und einer Dichte von 0,100 g/cm$^3$ aufweist. Dabei besteht eine erste Airlaidschicht aus einem behandeltem Fluff-Pulp, beispielsweise Biofluff TDR von Tembec Tartas, thermoplastischen Bikomponentenfasern, beispielsweise HC255 von Trevira oder Al-Bounce-Adhesion der Fa. FiberVisions und einem speziell für die Urin-Odor-Kontrolle entwickeltem SAP mit Naturextrakten. Auf der Oberseite der ersten Schicht ist eine zweite Airlaid-Schicht mit einem behandelten Fluff-Pulp, beispielsweise Tartas TDR und unbehandelter Fluff-Pulp, beispielsweise GP4881 der Fa. Georgia Pacific und der oben genannte SAP zur Urin-Odor-Kontrolle abgelegt worden. Auf der Oberseite der zweiten Airlaid-Schicht ist eine dritte Schicht aus unbehandeltem Fluff Pulp, beispielsweise GP4881 und thermoplastische Bikomponentenfasern abgelegt worden.

Die Lagenstrukturen der Proben A und B können die folgenden typischen Bereiche bezüglich der Zusammensetzung der Schichten aufweisen:

Der Anteil an Tissue in der Probe A beträgt 3,9 Gew.-% bezogen auf das Gesamtgewicht der Lagenstruktur. Die erste Schicht 1 umfasst 42,9 Gew.-% Pulp, 7,1 Gew.-% Bikomponentenfasern und 50 Gew.-% SAP, jeweils bezogen auf das Gesamtgewicht der Schicht 1. Die erste Schicht 1 hat einen Anteil am Gesamtgewicht der Lagenstruktur von 34,6 Gew.-%. Die zweite Schicht 2 umfasst 39,3 Gew.-% Pulp und 61,7 Gew.-% SAP, jeweils bezogen auf das Gesamtgewicht der Schicht 2, wobei die zweite Schicht 2 45,1 Gew.-% Anteil an dem Gesamtgewicht der Lagenstruktur hat. Die dritte Schicht 3 umfasst 82,1 Gew.-% Pulp und 17,9 Gew.-% Bikomponentenfasern, ebenfalls jeweils bezogen auf das Gesamtgewicht dieser Schicht. An der Lagenstruktur hat die dritte Schicht 3 einen Anteil in Höhe von 13,8 Ges.-%. Bezogen auf das Gesamtgewicht weist die Lagenstruktur 1,3 Gew.-% Latex auf, jeweils oben und unten.

Demgemäß ist der Aufbau der Lagenstruktur B mit den Einzelanteilen der jeweiligen Schicht, die bezüglich der Schichten 1, 2 und 3 wiederum jeweils auf das Gesamtgewicht jeder Einzelschicht bezogen sind, wie folgt:

- Tissue: 3,0 Gew.-%, bezogen auf das Gesamtgewicht der Lagenstruktur B;
- Schicht 1 mit 42,8 Gew.-% Pulp, 7,2 Gew.-% Bikomponentenfasern und 50 Gew.-% SAP;
- Schicht 2 mit 43,1 Gew.-% Pulp und 56,9 Gew.-% SAP;
- Schicht 3 mit 75,7 Gew.-% Pulp und 24,3 Gew.-% Bikomponentenfasern.

Die Schicht 1 hat 34,6 Gew.-%, die Schicht 2 hat 49,0 Gew.-% und die Schicht 3 hat 10,4 Gew.-% Anteil am Gesamtgewicht der Lagenstruktur. Des weiteren werden 1,3 Gew.-% Latex oben und unten an der Lagenstruktur verwandt.

Das Latex ist in folgender Form zum Einsatz gebracht worden: es ist eine Wasserbasierte EVA-Latex-Mischung genutzt worden, bei der der Latex-Anteil 16,0 Gew.-% und der Wasseranteil 84,0 Gew.-% beträgt. Diese Dispersion ist bei den Lagenstrukturen gemäß Proben A und B bzw. VH460.103 und VH600.101 zum Einsatz gelangt.

Die Bikomponentenfasern weisen einen Titer von beispielsweise 2,2 dtex und Faserlängen von etwa 3mm auf. Der Kern umfasst PET, während der Mantel ein Copolyolefin oder Polyethylen umfasst. Als Superabsorber wurde Favor Z 3269 der Fa. Stockhausen,Inc. verwendet. Als Latex-Bindemittel kam auf den Oberflächen der Lagenstruktur Ethylen-Vinylacetat (EVA)-Latex, beispielsweise Airflex®192 mit der Bezeichnung Vinnapas®192 der Fa. Wacker Chemie AG mit ~1.3 % Trockenrückstand nach der Härtung der Wasser-Latex-Dispersion zum Einsatz. Einen Überblick über die verwendeten Rohstoffe zur Herstellung der Proben VH460.103/Probe A und VH600.101/Probe B sowie deren Zusammensetzung geben die Tabellen 1 und 2.

Die Herstellung der Lagenstrukturen A und B wurde in folgenden Schritten vollzogen:

Die Airlaid-Lagen wurden als drei aufeinanderfolgende Schichten auf dem nassgelegten Tissue, welches als Trägermaterial dient, abgelegt und ein endloses Vlies geformt. Anschließend erfolgte eine Verdichtung der Lagenstruktur in einem Walzenspalt, der aus beheizten Glattwalzen gebildet worden ist. Anschließend erfolgte eine beidseitige Besprühung der Lagenstruktur mit einem Dispersionsbinder, das Ausbringen des Wassers, die Vernetzung des Latex und die Aufschmelzung des niedrigschmelzenden Mantels der Bikomponentenfaser in einem mehrstufigen Trocknungssystem. Nach der Erwärmung und Konsolidierung der Lagenstruktur erfolgt die Einstellung der erforderlichen Dicke in einem Spalt eines Glattwalzenkalanders.

Die an den Proben A, VH460.103, und B, VH600.101, sowie den Vergleichsproben MT410.104 und VE500.200 ermittelten Messergebnisse sind in der Tabelle 3 für die Dicke und in Tabelle 4 für mechanische Eigenschaften dargestellt.

Bei den im Folgenden aufgeführten WSP (Worldwide Strategic Partners)-Methoden handelt es sich um vereinheitlichte Standard-Test-Methoden der europäischen Vliesstoff-Organisation Edana und der amerikanischen Vliesstoff-Organisation Inda.

Bestimmung der Dicke:

Die Bestimmung der Dicke erfolgte nach der Standard-Test-Methode WSP 120.6 (05) für die Proben A, VH460.103, und B, VH600.101, sowie den Vergleichsproben MT410.104 und VE500.200 an jeweils 10 Einzelproben mit einer Abmessung von jeweils 7 cm x 7 cm. Dabei wurden jeweils Proben im trockenen Zustand, im getränkten Zustand mit 10 g Flüssigkeit/g Probe und im gesättigten Zustand gemessen. Die gemessenen Werte in Tabelle 3 belegen, dass beispielsweise das erfindungsgemäße Material VH600.101 mit einer Dichte von etwa 0,1 g/cm$^3$ im trockenen Zustand sehr locker aufgebaut ist, wodurch mehr freies Volumen für eine Flüssigkeitsaufnahme zur Verfügung steht und somit seine Dicke im nassen Zustand nicht so stark zunimmt, wie etwa die Dicke des Vergleichsmaterials VE500.200, welches im trocken Zustand stärker verdichtet ist, als das Material VH600.101. Somit ist die Dicke der vorgeschlagenen Lagenstruktur während der Flüssigkeitsaufnahme ziemlich gleichbleibend, was als Komfort-Eigenschaft für eine Benutzung betrachtet werden kann.

Bestimmung der Beugelänge und Biegsteifigkeit:

Zur Ermittlung der Biegesteifigkeit wurde die Standard-Test-Methode WSP 90.5 (05) herangezogen. Hierzu wurden die Proben in rechteckige Streifen geschnitten, deren Beugelänge an den vier Seiten der Streifen gemessen und deren Mittelwert gebildet. Zur Bestimmung der Biegesteifigkeit in mN*cm wurde die ermittelte Beugelänge im cm mit dem jeweiligen Flächengewicht der Probe multipliziert und durch 1000 dividiert. Der Tabelle 4 ist zu entnehmen, dass die Werte für die Beugelängen der Proben im nassen Zustand nur noch etwa 30% bis 35% der Werte des trockenen Zustands der Proben aufweisen. Die Steifigkeit der Proben im nassen Zustand weist hingegen nur noch etwa 5% der Steifigkeit des trockenen Zustands auf. Die Vergleichsproben MT410.104 und VE500.200 zeigen bezüglich dieser Eigenschaft eine ähnliche Tendenz.

Bestimmung der Reißfestigkeit:

Die Reißfestigkeit wurde nach der Standard-Test-Methode WSP 110.4 (05), Option B unter Verwendung einer Zwick-Prüfmaschine mit einem Klammerabstand von 200 mm und einem Vortrieb von 100 mm/min bestimmt. Gemäß Tabelle 4 sinkt die Reißfestigkeit der Proben im Nasszustand auf etwa 25% des Wertes, der im trockenen Zustand an dieser Probe ermittelt werden konnte (VH600.101) und auf etwa 20% des Wertes im Falle der Probe VH460.103. Die Vergleichsproben MT410.104 und VE500.200 zeigen einen geringeren Abfall der Reißfestigkeit vom trockenen zum nassen Zustand der Proben hin. Zudem sind der Tabelle 4 die Reißfestigkeiten für die Probe VH600.101 zu entnehmen, die an der kompletten Probe, dem Tissue und der oberen Seite der Probe, die die Flüssigkeitsaufnahmeschicht bildet, zu entnehmen. Dabei weist die komplette Probe höhere Werte für die Reißfestigkeit auf als die Vergleichsproben. Hier findet sich der Hinweis, dass die Außenlagen den wesentlichen Anteil für die Reißfestigkeit erbringen.

Bestimmung der Bindungsstärke:

Zur Bestimmung der Bindungsstärke gemäß WSP401.0, dass heißt der Kraft, die zum Auseinanderreißen der Lagen erforderlich ist, wurden 25 mm breite Proben bereitgestellt und an einer Seite etwa 3 cm aufgerissen. Anschließend sind an deren Außenlagen die Klammern befestigt worden. Hierbei wurden Proben mit 10 g Flüssigkeit/g Probe getränkt. Es wurden etwa 3 g schwere Proben mit 30 ml 0,9 %-iger NaCl-Lösung gleichmäßig getränkt und etwa 4 g schwere Proben mit 40 ml 0,9 %-iger Kochsalz-Lösung gleichmäßig getränkt und anschließend zügig gemessen, so dass die Messung der Proben im feuchten Zustand erfolgte. Die noch nicht aufgerissene Seite der Proben wurden unterstützend gehalten, dass heißt, das Material wurde immer auf der halben Höhe des Klammerabstands händisch gestützt, so dass das hohe Eigengewicht der Probe die Messung nicht verfälscht hat. Die Tabelle 4 zeigt, dass sich die gemessenen Werte vom trockenen zum nassen Zustand hin kaum ändern. Im Gegensatz hierzu weisen die Vergleichsproben MT410.104 und VE500.200 im nassen Zustand erheblich geringere Bindungsstärken als im trockenen Zustand auf.

Es wird angenommen, dass die Gleitwirkung der Flüssigkeitsspeicherschicht, die eine Entkoppelung der Schichten der Lagenstruktur bewirkt, in der Lage ist, ein gewisses Maß an Scher- und Zugkräften aufzunehmen und somit einem Zerreißen oder einer Delamination der Probe entgegenwirken kann.

Bestimmung der Flüssigkeitsaufnahmekapazität:

Die Flüssigkeitsaufnahmekapazität wurden nach der Standard-Test-Methode WSP 10.1 (05), Pos. 7.2 ermittelt. Hierbei wurden jeweils 5 Einzelproben mit einer Abmessung von 10 cm x 10 cm verwendet. Dementsprechend wurden Einzelproben von etwa 6 g der Probe VH600.101 hergestellt. Die Proben wurden vorbehandelt, indem sie 1 Minute in 0,9 %-ige NaCl-Lösung gelegt worden sind und anschließend 2 Minuten vertikal hängend abtropfen konnten.

Parallel hierzu wurde für SAP-haltige Materialien eine von der Firma Concert entwickelte Methode "CG Test 4 Rev.5 Prüfvorschrift für die Absorptionskapazität" vom 02.03.2009 herangezogen. Diese liefert abweichende Werte im Vergleich zu den Werten, die nach der Standard-Test-Methode WSP10.1 (05) ermittelt worden sind. Bei der von Concert entwickelten Methode werden die Proben 10 Minuten lang getaucht und tropfen 10 Sekunden vertikal ab. Die Anwendung der Concert-Methode führt zu höheren Werte für die Flüssigkeitsaufnahmekapazität der Proben, die im Bereich von über 20 g/g liegen können, als dieses bei der oben erwähnten Standard-Test-Methode gegeben ist, da der Superabsorber zwar schnell in der Lage ist, Flüssigkeit zu binden, jedoch seine ganze Saugkraft nicht innerhalb von einer Minute entfalten kann. In der Tabelle 4 sind die ermittelten Massen der jeweiligen Proben aufgeführt und deren Mittelwert berechnet worden.

Die nach der Vergleichmethode von Concert ermittelten Werte liegen über den Werten der Standard-Test-Methode.

Die Flüssigkeitsaufnahmekapazität LAC in % wird gemäß Standard-Test-Methode WSP 10.1 (05) nach folgender Formel berechnet:

$$LAC \% = \frac{M_n - M_k}{M_k} \times 100 \%$$

wobei $M_n$ die Masse der trockenen Probe und $M_k$ die Masse der nassen Probe ist.

Demnach weist beispielsweise die Probe VH600.101 eine Flüssigkeitsaufnahmekapazität von etwa 1763 % oder 17,63 g/g auf. Es wird deutlich, dass die erfindungsgemäßen Materialien eine höhere Flüssigkeitsaufnahmekapazität als die Vergleichsproben MT410.104 beziehungsweise VE500.200 aufweisen. Dieser Effekt wird auf Grund der fehlenden Bindefasern mit einer größeren Beweglichkeit der SAP-Partikel oder SAP-Fasern sowie der Cellulosefasern der Flüssigkeitsaufnahmeschicht erklärt, wodurch bei Flüssigkeitsaufnahme und damit verbundenen Quellvorgängen mehr freies Volumen für Flüssigkeit durch die vorhandene Beweglichkeit der Partikel gegeben ist.

Bestimmung der Scherfestigkeit:

Die Messungen zur Bestimmung der Scherfestigkeit wurden analog der Reißfestigkeits-Messmethode durchgeführt. Dabei wird als Scherung die Art der Verformung eines Körpers unter Einwirkung einer Kraft verstanden, wobei die Kraft gegen-parallel zu parallelen inneren oder äußeren Flächen eines Körpers wirkt. Die Flächen werden dabei relativ zueinander verschoben. Für die Messung der Scherfestigkeit sind aus den zuvor genannten erfindungsgemäßen Proben und Vergleichsproben jeweils 25mm breite Streifen mit einer Länge von über 20 cm, typischerweise 26 cm ausgeschnitten worden. Dabei wurden alle Materialien in Maschinenrichtung jeweils auf Länge geschnitten. Anschließend wurde diese Proben an den Außenflächen der Lagen ergriffen und etwa 3 cm aufgerissen. Dieses erfolgte an beiden Enden der Streifen.

Zuerst wurden die Proben ohne die Verwendung eines Tapes jeweils so eingespannt, dass die eine geöffnete Außenlage, zum Beispiel die Materialoberseite in der oberen Klammer eingespannt war und die andere Außenlage, das heißt die Materialunterseite unterhalb der oberen Einspannklammer lose verblieb. Danach wurde die geöffnete Außenlage, der dem oberen Ende gegenüberliegenden Seite, also die Materialunterseite in die untere Klammer eingespannt und die andere Außenlage, also die Materialoberseite verblieb lose oberhalb der unteren Einspannklammer.

Erwartungsgemäß war der Zusammenhalt bei den erfindungsgemäßen Proben VH 460.103 und VH 600.101 in der Innenlage aus Pulp und SAP nicht so hoch wie die Reißfestigkeit der Außenlagen, dass heißt der Tissue-unterstützten Pulp-SAP-Bikomponentenunterseite, verstärkt mit ausgehärtetem Latexbinder. Bei der Probe VH600.101 beträgt die Reißfestigkeit auf der Seite, an der das Tissue angeordnet ist, etwa ¾ der gemessenen Gesamtreißfestigkeit und die Reißfestigkeit der Oberseite der Lagenstruktur, die eine Pulp-Bikomponentenschicht mit ausgehärtetem Latexbinder aufweist liefert etwa ¼ der Gesamtreißfestigkeit, während die Innenlage, die keine Bindefasern aufweist, somit kaum einen Beitrag zur Reißfestigkeit leistet. Es wurden kaum Unterschiede zwischen Messungen mit beziehungsweise ohne Tape festgestellt.

Um die Vergleichsmaterialien ebenfalls messen zu können, war das Anbringen von Tapes notwendig, da an diesen Proben die geöffneten Außenlagen bereits beim Einspannen und Anfahren nach der oben genannten Methode vorzeitig abrissen. Durch das Aufbringen des Tapes auf die Ober- und Unterseite der Proben wurde das vorzeitige Abreißen der Außenlagen unterbunden, da die Reißfestigkeit des Tapes mit 45 N/cm oberhalb der Scherbruchkraft der Materialien liegt und die Außenlagen somit wirksam gegen Abriss fixiert worden sind.

Das Beklebens der Proben mit tesapack® 4024 PP, Variante braun wurde in folgenden Schritten vorgenommen:

Zuerst wurden Proben in einer üblichen A4-Handmustergröße in Längsrichtung mit einem 5cm breiten Tape beidseitig derart beklebt, dass das Tape mit der Oberfläche der Probe eine haftende Bindung einging. Danach wurden die 25mm breiten Streifen analog zu den zuvor genannten mechanischen Standardmessmethoden für die Reißfestigkeit, Biegsteifigkeit und Bindestärke mit einer Standard-Schneidapparatur auf typischerweise 26cm Länge geschnitten. Anschließend wurde die nun von beiden Seiten mit Tape beklebten Proben in der Mitte geöffnet und etwa 3cm aufgerissen, wie dieses zu Messung Bindungsstärke nach WSP401.0 vorgesehen ist, jedoch erfolgte das Aufreißen an beiden Enden des Streifens. Ebenso, wie zuvor bei den Versuchen ohne die Anwendung von Tapes erfolgte nun das Einspannen wechselseitig an den gegenüberliegenden Enden, während das eine Ende innerhalb des Klammerabstands lose verblieb, so dass es keinen Beitrag zur Reißfestigkeit liefert und nur die Scherkraft der Mittellage bestimmt wird.

Bei den Vergleichmaterialien wurden hohe und zugleich ungünstige Werte für die Scherbruchkraft beobachtet. So lag der Wert für MT410.104 bei 37N, was auf die thermische Bindung der Pulp- und Bikomponentenfasern in Verbindung mit den SAP-Partikeln der Innenlage zurückgeführt wird. Für das Vergleichsmaterial VE500.200 wurde eine Scherkraft von 44N ermittelt, die auf eine hohe Dichte und Festigkeit, insbesondere auch im Inneren dieser Lagenstruktur zurückzuführen ist. Im Gegensatz hierzu konnten bei den erfindungsgemäßen Proben 5 - 6N für VH600.101 und 4N für VH460.103 ermittelt werden. Die Messungen zeigen, dass sich die erfindungsgemäßen Proben selbst im Trockenzustand leichter gegeneinander parallel verschieben lassen und somit im Inneren der Lagenstruktur den Vorteil der Beweglichkeit von reinen Pulp-SAP-Materialien bieten, wobei jedoch die Festigkeit in den Außenlagen zu dem gewünschte komfortablen, textilen Eigenschaftsbild führt.

Die Kombination aus freier Absorption und mechanischer Oberflächen-Festigkeit im nassen Zustand unterscheidet die erfindungsgemäße Lagenstruktur von rein mechanisch verdichteten Zellstoff-SAP-Airlaids, wie sie beispielsweise von McAirlaid's und Rayoniers EAM bekannt sind. Diese Lagenmaterialien besitzen keine ausreichende Nassintegrität und ähneln damit dem Verhalten von sogenannten "airfelt pads".

Im Vergleich zu homogen thermisch gebundenen Airlaid-Materialien, wie beispielsweise das Vergleichsmaterial MT410.104 ist die Deformation des erfindungsgemäßen Lagenmaterials im nassen Zustand reversibel, was eine geringere Steifigkeit der Lagenstruktur zur Folge hat. Zudem weist das Material durch große Poren in der Mitte der Lagenstruktur auch im trockenen Zustand eine hinreichende Flexibilität auf, das heißt die Cantilever-Biegesteife ist bei diesem Material auch im trockenen Zustand geringer als bei thermobondierten Produkten mit steifer Matrix.

Zugversuche zur Bestimmung der Scherfestigkeit:

In Fortführung der oben beschriebenen Versuche zur Bestimmung einer Scherfestigkeit beziehungsweise einer Scherbruchkraft für die Proben A (VH 460.103) und B (VH 600.101) und der Vergleichsproben MT 410.104 und VE 500.200 wurden weitere Versuche an präparierten Proben vorgenommen. Im Folgenden wird die Versuchsdurchführung vorab grob erläutert und anschiebend im Detail beschrieben. Eine Präparation der Proben beziehungsweise Airlaid-Vliesstoff-Materialien erfolgte dahingehend, dass sämtliche Proben beidseitig mit einem Sprühkleber besprüht wurden und nach einer Abtrockenzeit ebenfalls beidseitig mit einem Klebeband beklebt wurden. Die Beaufschlagung der Proben mit dem Sprühkleber gewährleistet eine permanente Verbindung zwischen der Probe und dem Klebeband, wobei eine permanente Verbindung auch im nassen Zustand der Proben gewährleistet blieb. Derartig präparierte Proben ermöglichen daher eine Bestimmung einer Nassscherfestigkeit. Die Messungen zur Bestimmung der Nassscherfestigkeit wurden analog zur Reißfestigkeit-Messmethode durchgeführt. Dabei wird als Scherung die Art der Verformung eines Körpers unter Einwirkung einer Kraft verstanden, wobei die Kraft gegen parallel zu parallelen inneren oder äußeren Flächen eines Körpers wirkt. Die Flächen der Proben werden dabei relativ zueinander verschoben. Für die Messung der Scherfestigkeit sind aus den präparierten Airlaid-Vliesstoff-Materialien die Proben A und B und die Vergleichsproben mit einer Breite von jeweils 25 mm und einer Länge von über 20 cm typischerweise 26 cm ausgeschnitten worden. Dabei wurden alle Materialien in Maschinenrichtung jeweils auf Länge geschnitten, so dass die Proben als Streifen vorlagen.

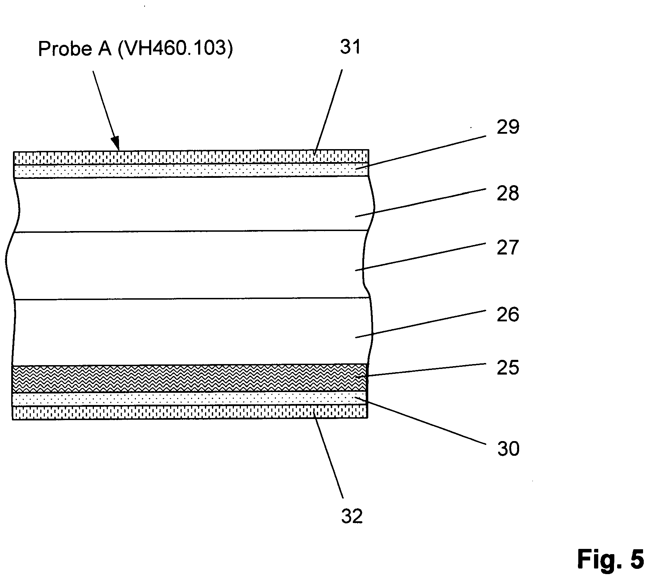

In der Fig. 5 ist die Probe A mit der Probenbezeichnung VH 460.103 in einem Schnitt und in einer Seitenansicht in Längsrichtung schematisch wiedergegeben. Die Probe A umfasst eine Tragschicht 25 aus einem Tissue-Material, auf der eine Flüssigkeitsaufnahmeschicht 26 abgelegt wurde. Die Flüssigkeitsaufnahmeschicht 26 ist aus behandeltem Fluff-Pulp, partikelförmigem SAP-Material und thermoplastischen Bikomponentenfaser gebildet und stellt eine erste Airlaid-Schicht dar. Auf die Flüssigkeitsaufnahmeschicht 26 ist eine Feuchtigkeitsspeicherschicht 27 ebenfalls als Airlaid-Schicht abgelegt worden. Die Flüssigkeitsspeicherschicht 27 umfasst hierbei behandeltes Fluff-Pulp und SAP-Partikel. Die Flüssigkeitsspeicherschicht 27 umfasst keine thermoplastischen Bikomponentenfasern. Über der Flüssigkeitsspeicherschicht 27 ist eine weitere Flüssigkeitsverteilungsschicht 28 abgelegt worden, die ebenfalls eine Airlaid-Schicht ist. Beidseitig wurde die Lagenstruktur mit einem Dispersionskleber, auch Latex-Bindemittel genannt, imprägniert. Der Binderauftrag aus Ethylen-Vinylacetat (EVA)-Latex, beispielsweise Airflex®192 mit der Bezeichnung Vinnapas®192 der Fa. Wacker Chemie AG erfolgt vollflächig, nach Trocknen und Aushärten der Wasser-Latex-Dispersion verbleiben ~1.3 % je Seite, dies entspricht bei 460 gsm 6 g je m$^2$ Trockenrückstand auf jeder Seite. Die Latex-Bindemittelschichten 29, 30 wurden als schraffierte Flächen dargestellt. Zu beachten ist hierbei, dass die Dickenverhältnisse der Lagen beziehungsweise Schichten nur skizziert sind. Beispielsweise weicht die Dicke der Latexschichten 29, 30 von den realen Gegebenheiten ab. So ist es insbesondere möglich, dass das Latexbindemittel lediglich auf der Flüssigkeitsverteilungsschicht 28 aufliegt und/oder bereichsweise in die Flüssigkeitsverteilungsschicht dispersiv eindringt und eine Bindemittelschicht in der Flüssigkeitsverteilungsschicht 28 bildet. Entsprechendes gilt ebenfalls für das auf die Tissue-Lage 25 aufgebrachte Latexbindemittel-Schicht 30, die ebenfalls schematisch stark vergrößert dargestellt ist. Insgesamt weist die Probe eine Dicke von etwa 5mm auf.

Die Probe A wurde für die Zugversuche der Scherfestigkeitswerte wie folgt präpariert. Die Probe A umfasst eine beidseitig mit einem Latex-Bindemittel imprägnierte Lagenstruktur aus Tissue 25, Flüssigkeitsaufnahmeschicht 26, Flüssigkeitsspeicherschicht 27 und Flüssigkeitsverteilungsschicht 28. Die Lagenstruktur wurde beidseitig mit Sprühkleber "UHU® 3-in-1" Sprühkleber, Dose 500ml, Artikelnummer UH48905, erhältlich im UHU® Profishop besprüht. Das Besprühen erfolgte beidseitig in je drei Sprühlinien, wonach eine Abtrocknen des Sprühklebers von zehn Minuten erfolgte. Als Sprühklebeform wurde die unter der Bezeichnung "UHU® 3-in-1, erste Variante, permanent" vertriebene permanent klebende Variante des Sprühklebers eingesetzt. Je Seite werden 3 waagerechte Sprühlinien nach Positionieren des Streifens mit der längeren Seite in waagerechter Ausrichtung, d.h. Sprühlinien im Abstand vom Airlaid aus 20-25cm Abstand, konform mit dem Verarbeitungshinweis auf der Sprühkleberdose, in der Mitte längs überlagernd waagerecht von links nach rechts, dann von rechts nach links und abschließend noch einmal von links nach rechts. Die einfache Dosierung je Fläche ist ca. 200ml/m$^2$, bei 3 übereinanderliegenden Sprühlinien werden so ca. 600ml/m$^2$ appliziert. Nach dem Abtrocknen wurde die Lagenstruktur beidseitig mit fünf Zentimeter breitem Klebeband 31, 32 beklebt. Als Klebeband wurde das Klebeband mit der Bezeichnung Tesapack® 4024 PP, Variante braun, einem PP-Film mit Acrylat-Klebmasse, erhältlich bei TESA SE, einem Beiersdorf Unternehmen verwendet. Um ein definiertes Walzgewicht zu erzielen, wurde die Probe A einmal mit einer 7,4 kg schweren Messingwalze gewalzt. Aus der derart präparierten Lagen-struktur wurden Proben mit einer Länge von 260 mm und einer Breite von 25 mm ausgeschnitten.

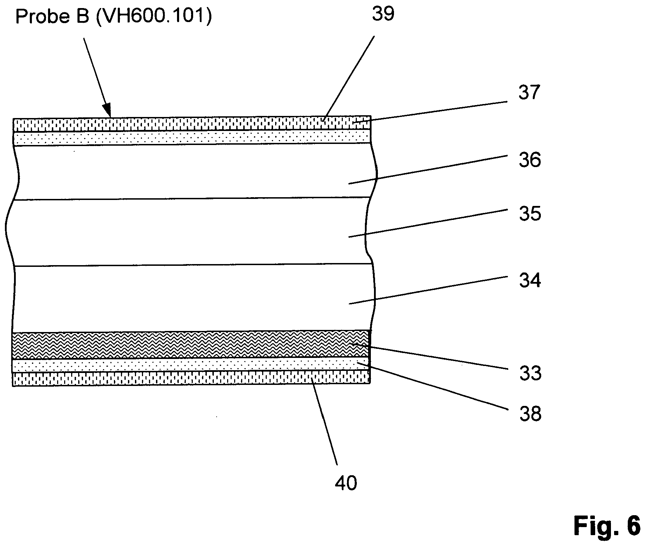

In der Fig. 6 ist ein schematischer Schnitt durch eine Probe B mit der Bezeichnung VH 600.101 im Querschnitt und in einer Seitenansicht wiedergegeben. Die Probe B weist eine Tragschicht 33 auf, die eine Tissue Lage ist und auf die eine Flüssigkeitsaufnahmeschicht 34 in einem Airlaid-Verfahren abgelegt wurde. Die Flüssigkeitsaufnahmeschicht 34 weist behandeltes Fluff-Pulp, SAP-Partikeln und thermoplastischen Bikomponentenfasern auf, vorzugsweise besteht die Flüssigkeitsaufnahmeschicht 34 aus diesen Komponenten, so wie hier für die Messung. Auf die Flüssigkeitsaufnahmeschicht 34 wurde eine Flüssigkeitsspeicherschicht 35 ebenfalls im Airlaid-Verfahren abgelegt. Die Flüssigkeitsspeicherschicht 35 weist behandeltes Fluff-Pulp und SAP-Partikeln auf, vorzugsweise besteht die Flüssigkeitsaufnahmeschicht 34 aus diesen Komponenten, so wie hier für die Messung. Oberhalb der Flüssigkeitsspeicherschicht wurde eine Flüssigkeitsverteilungsschicht 36 abgelegt, die aus unbehandeltem Fluffpulp und thermoplastische Bikomponentenfasern gebildet ist. Die Lagenstruktur aus Tragschicht 33, Flüssigkeitsaufnahmeschicht 34, Flüssigkeitsspeicherschicht 35 und Flüssigkeitsverteilungsschicht 36 wurde beidseitig mit einer Bindemittelschicht 37, 38 aus Latex versehen. Der Binderauftrag aus Ethylen-Vinylacetat (EVA)-Latex, beispielsweise Airflex®192 mit der Bezeichnung Vinnapas®192 der Fa. Wacker Chemie AG erfolgt vollflächig, nach Trocknen und Aushärten der Wasser-Latex-Dispersion verbleiben ~1.3 % je Seite, dies entspricht bei 600 gsm 7,8 g je m$^2$ Trockenrückstand auf jeder Seite. Zur Präparation der Probe B für die Zugversuche zur Bestimmung der Scherfestigkeit wurde die mit dem Bindemittel versehene Lagenstruktur wie bei Probe A mit Sprühkleber, der unter der Bezeichnung UHU 3-in-1, erste Variante, permanent vertrieben wird, besprüht. Aufgrund der vernachlässigbar geringen Dicke des Sprühklebers in Bezug auf die Lagenstruktur wurde die Sprühkleberschicht nicht dargestellt. Nach einem zehnminütigen Abtrocknen des Sprühklebers wurde die Lagenstruktur beidseitig mit Klebeband 39, 40 beklebt. Als Klebeband 39, 40 kam ebenfalls wie in der Probe A das Klebeband 39, 40 mit der Bezeichnung Tesapack® 4024 PP, Variante braun in einer Breite von 5 cm zum Einsatz. Nach dem Bekleben wurde die beklebte Lagen-struktur mit einer 7,4 kg schweren Messingwalze einmal gewalzt, um ein definiertes Gewicht zu erzielen. Aus dem präparierten Probenmaterial wurden 260 mm lange und 25 mm breite Proben für die Zugversuche ausgeschnitten.

Der Sprühkleber hält eine permanente Verbindung zwischen der mit Bindemittel versehenen Lagestruktur und dem Klebeband aufrecht. Dies sichert die Messung, das heißt, die Durchführung von Zugversuchen zur Bestimmung der Scherfestigkeit auch im nassen Zustand der Proben.

Wie vorstehend unter den Beispielen ausgeführt unterscheidet sich die Probe A von der Probe B in der Zusammensetzung der Lagen der Flüssigkeitsaufnahmeschicht, der Flüssigkeitsspeicherschicht und der Flüssigkeitsverteilungschicht. So wird insbesondere die Flüssigkeitsspeicherschicht der Probe A aus 39,3 Gew.-% Fluff-Pulp und 61,7 Gew.-% SAP-Partikeln gebildet, wohingegen die Flüssigkeitsspeicherschicht der Probe B aus 43,1 Gew.-% Fluffpulp und 56,9 Gew.-% SAP-Partikeln gebildet ist. Auch im Aufbau der Flüssigkeitsverteilungsschicht unterscheiden sich die Proben A und B. Weist die Flüssigkeitsverteilungsschicht 27 der Probe A einen Anteil von 82,1 Gew.-% Fluffpulp und 17,9 Gew.-% Bikomponentenfasern auf, so wird die Flüssigkeitsverteilungsschicht (36) der Probe B aus 75,7 Gew.-% Fluffpulp und 24,3 Gew.-% Bikomponentenfasern gebildet. Ein wesentlicher Unterschied zwischen den Proben A und B und den Vergleichsproben MT410.104 und VE500.200 besteht darin, dass die Proben A und B eine Flüssigkeitsspeicherschicht 27, 35 aufweisen, die keine Bikomponentenfasern aufweisen.

Die Fluff-Pulp-Zellulosefasern der Flüssigkeitsspeicherschicht sind nicht mit einem Bindemittel imprägniert, beziehungsweise weisen kein Bindemittel auf. Das Fehlen eines Bindemittels ermöglicht es hierbei, dass die in der Flüssigkeitsspeicherschicht 27, 35 enthaltenen SAP-Partikel nahezu ungehindert anschwellen können und/oder bei einer Beaufschlagung mit einer Scherkraft sich zueinander bewegen können, was der Flüssigkeitsspeicherschicht einen Freiheitsgrad einer Beweglichkeit einräumt.

Neben den in den Fig. 5 und Fig. 6 erläuterten Verfahren zur Präparation der Proben A und B wurden ebenfalls Proben C und D für Zugversuche eingesetzt, bei denen auf den Einsatz des Sprühklebers auf die Bindemittelschicht verzichtet wurde, die aber in den anderen Merkmalen mit den beschriebenen Proben A und B übereinstimmen.. Die eine Vergleichsprobe C (MT410.104) ist thermisch gebunden, d.h. ohne Verwendung eines Bindemittels wie Latex. Vielmehr werden die Eigenschaften von Bindefasern in Form von Bikomponentenfasern genutzt. Die zweite Vergleichsprobe D (VE500.200) nutzt hingegen ein Bindemittel in Form eines Dispersionsbinders enthaltend Latex.

Die Vergleichsmaterialien umfassen im wesentlichen ebenfalls drei Schichten:

Das Material MT410.104 weist nach einem Kalandrierschritt ein Flächengewicht von 410 g/m$^2$ bei einer Dicke von etwa 5,4 mm (bei 0,5 kPa) auf. Dabei weist eine erste Schicht eine Airlaidschicht aus einem unbehandeltem Fluff-Pulp, beispielsweise NB416 von Weyerhaeuser, mit thermoplastischen Bikomponentenfasern, beispielsweise HC255 von Trevira, auf. Auf der Oberseite der ersten Schicht ist eine zweite Schicht in Form einer Airlaid-Schicht mit einem unbehandelten Fluff-Pulp, beispielsweise NB416 von Weyerhaeuser, und einem Superabsorber, beispielsweise Favor von Evonik Stockhausen, abgelegt worden. Auf der Oberseite der zweiten Schicht ist eine dritte Schicht aus unbehandeltem Fluff Pulp, beispielsweise NB416 von Weyerhaeuser und thermoplastische Bikomponentenfasern, beispielsweise HC255 von Trevira, abgelegt worden.

Die Lagenstrukturen können die folgenden typischen Bereiche bezüglich der Zusammensetzung der Schichten aufweisen:

Die Schicht 1 umfasst 82,8 Gew.-% Pulp und 17,1 Gew.-% Bikomponentenfasern, jeweils bezogen auf das Gesamtgewicht der Schicht. Die zweite Schicht umfasst 39,1 Gew.-% Pulp, 9,7 Gew.-% Bikomponentenfasern und 51,2 Gew.-% SAP, jeweils bezogen auf das Gesamtgewicht der Schicht. Die dritte Schicht umfasst 74,4 Gew.-% Pulp und 25,6 Gew.-% Bikomponentenfasern, ebenfalls jeweils bezogen auf das Gesamtgewicht dieser Schicht. Die Schichtenanteile am Gesamtgewicht der Lagenstruktur sind wie folgt: Schicht 1 29,2 Gew.-% ; Schicht 2 51,3 Gew.-% ; Schicht 3 19,5 Gew.-% .

Die Werte und Zusammensetzungen für das Material VE500.200 sind hingegen wie folgt:

Das Material VE500.200 weist nach Durchlauf durch einen Embosser mit speziellem Prägemuster und Kalandrierschritt ein Flächengewicht von 500 g/m$^2$ bei einer Dicke von etwa 1,6 mm (bei 0,5 kPa) auf. Dabei weist eine erste Schicht eine Airlaidschicht aus einem behandeltem Fluff-Pulp, beispielsweise GP4821 von Georgia Pacific oder Biofluff TDR von Tembec Tartas auf. Auf der Oberseite der ersten Schicht ist eine zweite Schicht umfassend eine Airlaid-Schicht mit einem unbehandelten Fluff-Pulp, beispielsweise NB416 von Weyerhaeuser, und Superabsorber, beispielsweise EK-X EN52 von Ekotec, abgelegt worden. Auf der Oberseite der zweiten Schicht ist eine dritte Schicht aus behandeltem Fluff Pulp, beispielsweise GP4821 von Georgia Pacific oder Biofluff TDR von Tembec Tartas abgelegt worden. Nach der Formung dieser Lagenstruktur erfolgte eine Besprühung der beiden äußeren Oberflächen der Lagenstruktur mittels eines Dispersionsbinders aus einer Wasser-basierten EVA-Latex-Mischung, wobei ein Latex-Anteil 4,5 Gew.-% und ein Wasseranteil 95,5 Gew.-% betrug.

Demgemäß ist der Aufbau der Vergleichsprobe VE500.200 mit den Einzelanteilen der jeweiligen Schicht, die wiederum jeweils auf das Gesamtgewicht jeder Einzelschicht bezogen sind, wie folgt:

- - Schicht 1 mit 100 Gew.-% Pulp,
- - Schicht 2 mit 25,3 Gew.-% Pulp und 74,7 Gew.-% SAP;
- - Schicht 3 mit 100 Gew.-% Pulp.

Die Schichtenanteile am Gesamtgewicht der Lagenstruktur sind wie folgt: Schicht 1 13,8 Gew.-% ; Schicht 2 65,9 Gew.-% ; Schicht 3 16,3 Gew.-% , neben jeweils 2,0 Gew.-% Latex Trockenrückstand oben und unten, nachdem der Wasseranteil nicht mehr vorhanden ist.

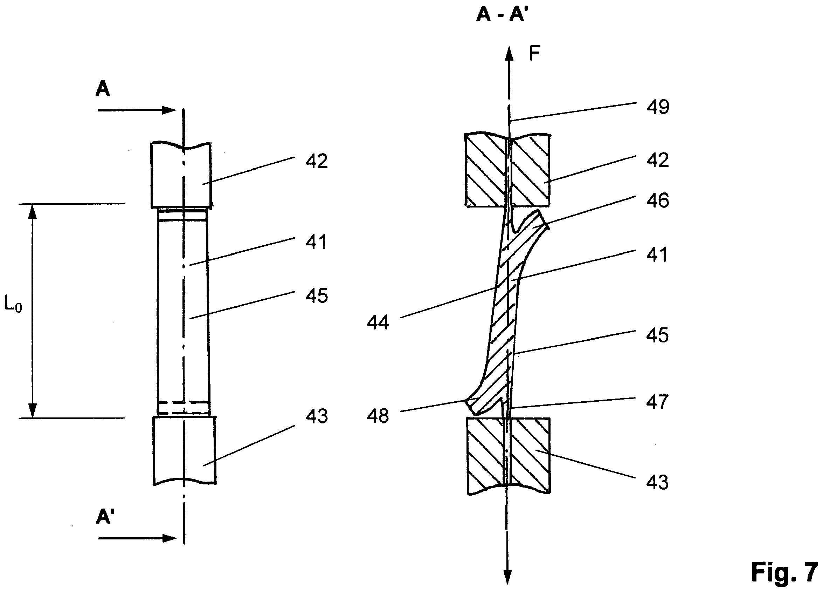

In der Fig. 7 ist eine prinzipielle Darstellung einer Einspannung einer Probe 41 zwischen Spannbacken 42, 43 einer Messapparatur für Zugversuche. Als Messapparatur wurde dabei eine Maschine der Firma Zwick aus Ulm mit der Bezeichnung Zwick/Roehl mit einem Längenänderungsaufnehmer Typ BZ 2,5/PN1S eingesetzt. Es handelt sich hierbei von der Bauweise her um eine Maschine zur Aufnahme eines Spannungs-Dehnungsverlaufs, im Modus constant-rate-of-Extention (CRE).

Grundlage der Versuche bildete die standardisierte Messmethode zur Ermittlung einer Bruch- und/oder Zugkraft und Dehnung für nicht gewebte Materialien. Der standardisierte Test ist mit WSP 110.4 (05) bezeichnet. Der Standardtest bezeichnet einen Test zur Bestimmung einer Dehnung und einer Zugkraft bei nicht gewebten Materialien und ist auch als "Standard Test Method for Breaking Force and Elongation of Nonwoven Materials (Strip Method)" bezeichenbar.. Es wird im CRE (constant-rate-of-extension)-Modus gemessen, das heißt die Vergrößerung der Längenrate der Probe ist proportional mit der Zeit. Es wird allgemein die Option B mit folgenden Änderungen ausgewählt. Die in dem Standardtest unter der Option B verwendeten Proben oder Prüfteile weisen eine Breite von 50 mm und eine Länge von 200mm auf. Im Unterschied zum standardisierten Test wurden für die Zugversuche Proben von 25 mm Breite und einer Länge zwischen den Spannbacken von $L_0$=200 mm eingesetzt, das heißt, im Zugversuch betrug die Einspannlänge 200 mm. Darüber hinaus wurde mit einer Vorspannkraft von 0,5 N und einer Prüfgeschwindigkeit von 100 mm/min geprüft. Geprüft wurde unter Laborbedingungen bei 23 °C und 50 % relativer Luftfeuchte zuzüglich der üblich zugelassenen Toleranzen. Die Einspannlänge $L_0$=200 mm ist in der Fig. 7 in der Draufsicht auf die Spannbacken 42, 43 und die Probe 41 eingetragen. Die Breite der Spannbacken beträgt 60 mm, so daß die Proben mit ihrer Breite von 25 mm bei mittiger Einspannung gut aufgenommen werden.

Die rechte Darstellung in der Fig. 7 zeigt einen schematischen Schnitt entlang der Linie A-A' und verdeutlicht die Einspannung der Probe 41 zwischen den oberen und unteren Spannbacken 42, 43 der Messapparatur. Die Proben A und B sowie die Vergleichsproben MT 410.104 und VE 500.200 wurden alle in Maschinenrichtung jeweils auf Länge geschnitten. Anschließend wurden die Proben an den Außenflächen 44, 45 der Lagen ergriffen und etwa 3 cm mittig dickenhalbierend aufgerissen. Dieses erfolgte an beiden Enden der Proben 41.

Wie in der rechten Abbildung der Fig. 7 dargestellt wurden die gelösten, mit Klebeband versehenen Außenlagen jeweils so eingespannt, dass die eine geöffnete Außenlage 44, zum Beispiel die Materialoberseite in der oberen Spannbacke 42 eingespannt war und die andere Außenlage 46, das heißt die Materialunterseite unterhalb der oberen Spannbacke 42 lose verblieb. Danach wurde die geöffnete Außenlage 47 der dem oberen Ende gegenüberliegenden Seite, also die Materialunterseite 45 in die untere Spannbacke 43 eingespannt und die andere Außenlage 48, also die Materialoberseite 44 verblieb lose oberhalb der unteren Spannbacke 43. Dargestellt ist der eingespannte Zustand einer Probe 41 im unbelasteten Zustand vor dem Beginn der Zugprüfung. Wie deutlich zu erkennen ist, und durch die Symmetrielinie 49 veranschaulicht erfährt die Probe 41 im Bereich der Flüssigkeitsaufnahmeschicht, der Flüssigkeitsspeicherschicht und der Flüssigkeitsverteilungsschicht eine Scherbeanspruchung. Folgende Versuchsreihen wurden durchgeführt.

In einer ersten Versuchsreihe wurden die Proben 41 im trockenen Zustand eingespannt und mit einer Zugkraft in Richtung der Pfeile F beaufschlagt. Die Versuche wurden 3 mal durchgeführt.

In einer zweiten Versuchsreihe wurden wiederum drei Zugversuche durchgeführt, wobei die Proben 41 durch ein einminütiges Tauchen in eine 0,9 %-ige NaCl-Lösung präpariert wurden. Nach dem Tauchen konnten die Proben 41 zwei Minuten lang senkrecht abtropfen, was wiederum der standardisierten Testmethode WSP 10.1, Absorption entspricht.

In einer dritten Zugversuchsreihe wurden ebenfalls drei Zugversuche durchgeführt. Dabei wurden die Proben 41 zehn Minuten lang in eine 0,9 %-ige NaCl-Lösung getaucht und anschließend für zehn Sekunden senkrecht abtropfen gelassen. Dies entspricht dem Concert CG-Test 4 Rev. 5 Prüfvorschrift für die Absorptionskapazität vom 02.03.2009.

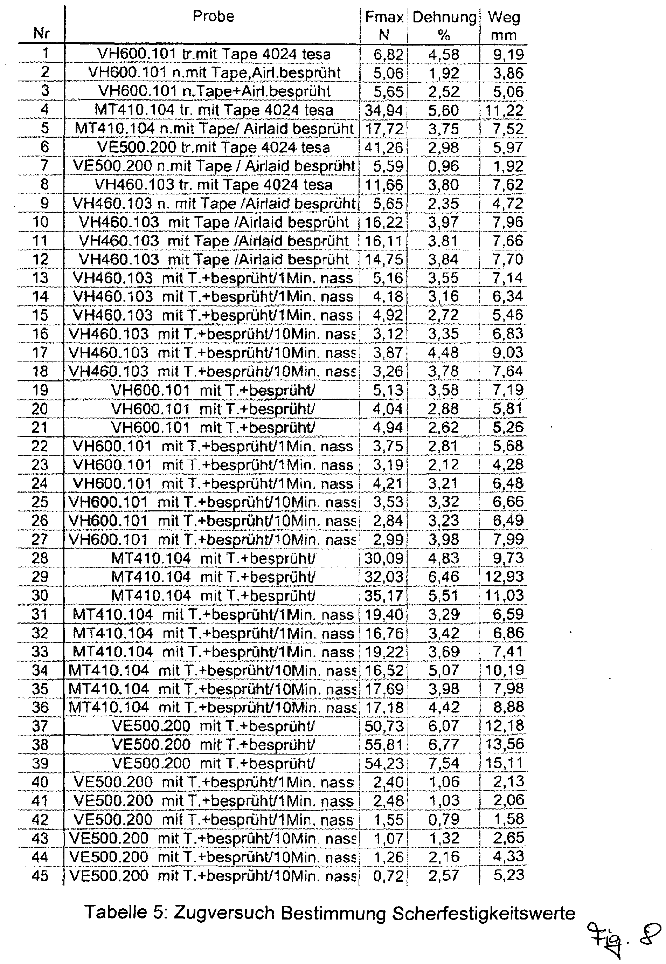

In der Fig. 8 sind die Ergebnisse der Zugversuche zur Bestimmung einer Scherfestigkeit als Tabelle 5 wiedergegeben. Die Versuchsergebnisse 1 bis 9 zeigen Versuche mit den Proben C und D sowie der Vergleichsproben MT 410.104$_{ohne}$ und VE 500.200$_{ohne}$, die mit einem Klebeband (Tape) an den Außenlagen verstärkt wurden, die Außenlagen aber nicht mit Sprühkleber besprüht waren. Die Bezeichnung tr. bezeichnet hierbei Proben im trockenen Zustand und die Bezeichnung n. steht für Proben im nassen Zustand.

Die Versuche mit der laufenden Nummer 10 bis 45 zeigen die Versuche auf, die im trockenen Zustand, nach einminütiger Befeuchtung und nach zehnminütiger Befeuchtung durchgeführt wurden.

Die gemäß der Erfindung ausgebildeten Proben A, C (VH 460.103) und B, D (VH 600.101) wurden den Vergleichsproben MT 410.104, MT 410.104$_{ohne}$ und VE 500.200, VE 500.200$_{ohne}$ gegenüber gestellt. Die Zugversuche sind fortlaufend von 1 bis 45 nummeriert. Der Zugversuch mit der Nummer 1 beschreibt einen Zugversuch mit einer gemäß der Erfindung ausgebildeten Probe D im trockenen Zustand, wobei die Probe D mit Klebeband an den Außenflächen beklebt war. Die Probe wurde wie vorstehend beschrieben und in der Fig. 7 dargestellt, in die Messapparatur eingespannt und mit einer Kraft F beaufschlagt. Durch das Einspannen der gegenüberliegenden Außenlagen konnte mit dem Zugversuch ein Scherfestigkeitswert der Lagenstruktur bestimmt werden. Der maximale Scherfestigkeitswert für die Probe D im Versuch Nummer 1 lag bei F max = 6,82 N / 25 mm bei einem Verfahrweg der Spannbacken von insgesamt 9,19 mm, was einer Dehnung von 4,58 % bezogen auf die Einspannlänge $L_0$ entspricht. Hierbei rechnet sich die Dehnung aus dem Verhältnis:

$$\text{Dehnung} = \Delta L / L_0 \text{ [in \%]}.$$

Hierbei entspricht $\Delta L$ dem Weg, den die Spannbacken bis zum Erreichen der Maximalkraft und dem letztendlichen Zerreißen der Probe zurücklegen.

Der Zugversuch mit der laufenden Nummer 2 wurde mit einer nassen Probe D durchgeführt, wobei hier ein Beträufeln der Probe D in die Seiten hinein vorgenommen wurde. Ein Maß für die Befeuchtung war hierbei das Eigengewicht der Probe D, wobei 10 ml einer 0,9 %-igen NaCl-Lösung je Gramm Eigengewicht der Probe zugeführt wurden. So wurde der Probe D gemäß dem Zugversuch Nr. 2, 40 ml 0,9 %-ige NaCl-Lösung zugeführt. Die im Zugversuch Nummer 1 ermittelte geringe Scherfestigkeit von F max = 6,82 N wurde durch das Befeuchten der Probe verringert, wobei im Zugversuch Nummer 2 ebenfalls eine geringe Scherfestigkeit von F max$_1$ = 5,06 N ermittelt wurde. Auch der Zugversuch Nummer 3 für die Probe D, der ebenfalls im nassen Zustand durchgeführt wurde, bestätigt die geringe Scherfestigkeit, die mit einem Wert von F max$_3$ = 5,65 N ebenfalls sehr gering ausfällt. Die Messungen der Zugversuche Nummer 1 bis 3 zeigen, dass sich die erfindungsgemäßen Proben im trockenen wie im nassen Zustand sehr leicht gegeneinander parallel verschieben lassen.

Der Wert der Scherfestigkeit bezieht sich bei allen Proben auf 25 mm Probenbreite.

Die Probe C, die im Zugversuch Nummer 8 im trockenen Zustand untersucht wurde, wies eine maximale Scherfestigkeit von F max$_8$ = 11,66 N auf. Dieser ebenfalls geringe Wert wurde im nassen Zustand gemäß dem Zugversuch Nummer 9 nahezu halbiert auf ein F max$_8$ = 5,65 N. Zu beachten ist ebenfalls, dass auch der zurückgelegte Weg zur Erreichung der maximalen Zugkraft für die Probe B mehr als halbiert und für die Probe A nahezu halbiert wurde. Die gemäß der Erfindung ausgebildeten Proben C und D weisen eine geringe Scherfestigkeit auf, was wiederum einem Vorteil in der Beweglichkeit der Lagen zueinander entspricht.

Im Gegensatz zu den erfindungsgemäß aufgebauten Proben C und D weisen die Vergleichsproben gemäß den Zugversuchen Nummer 4 bis 7 eine sehr hohe Scherfestigkeit von F max$_4$ = 34,94 N und F max$_6$ = 41,26 N auf. In Bezug auf eine Beweglichkeit bedeutet dies, dass sehr hohe Kräfte erforderlich sind, um die Lagenstruktur der Vergleichsproben gegeneinander zu verschieben. Weist die Vergleichsprobe MT 410.104 im nassen Zustand immer noch eine hohe Scherfestigkeit auf, so sinkt die Scherfestigkeit der Vergleichsprobe VE 500.200$_{ohne}$ im nassen Zustand (Zugversuch Nummer 7) deutlich ab und liegt mit F max$_7$ = 5,59 im Bereich der erfindungsgemäßen Proben A und B. Das Vergleichsmaterial VE 500.200$_{ohne}$ enthält keine Schmelzklebefasern zur Nassverbindung und der Oberflächenbinder wirkt nicht in der Innenlage. Das Absorptionsmaterial hat somit die Möglichkeit aufzuquellen und ermöglicht eine leichte Verschiebung der Lagen gegeneinander. Die fehlenden Schmelzklebefasern in der Vergleichsprobe VE 500.200$_{ohne}$ wirken sich positiv auf eine Beweglichkeit der Lagen zueinander aus, so dass ein Verschieben der Lagen zueinander in nassem Zustand gewährleistet werden kann.

Die in der Tabelle 5 der Fig. 8 dargestellten Zugversuche Nummer 10 bis 18 zeigen die Ergebnisse der Zugversuche in trockenem sowie in nassen Zustand der erfindungsgemäßen Probe A. Die Proben wurden mit Sprühkleber besprüht und mit Klebeband zur Verstärkung der Außenlage versehen. Weisen die in trockenem Zustand durchgeführten Zugversuche Nummer 10, 11 und 12 eine in Bezug auf den ohne Sprühkleber ausgeführten Versuch Nummer 8 eine höhere Scherfestigkeit auf, so kann dieser leicht erhöhte Scherfestigkeitswert auch auf den Zustand und den Einsatz des Sprühklebers zurückgeführt werden. Im nassen Zustand hingegen fällt der Scherfestigkeitswert wieder signifikant ab und liegt mit Werten zwischen F max$_{13}$ = 5,16 N und F max$_{14}$ = 4,18 N noch unter den Werten des Zugversuchs Nummer 9 ohne Sprühkleber. Wird die Probe A, wie in den Versuchen 16, 17 und 18 aufgezeigt über eine längere Zeit einer Befeuchtung ausgesetzt, so fallen die Scherfestigkeitswerte noch einmal deutlich ab.

Wird die Probe A wie in den Zugversuchsnummern 16 bis 18 vorgenommen für 10 Minuten in eine 0,9 NaCl-Lösung getaucht, so haben die SAP-Partikel ausreichend Zeit um Feuchtigkeit aufzunehmen und zu quellen. Aufgrund der fehlenden Bindefasern in der Flüssigkeitsspeicherschicht kann das Superabsorbermaterial (SAP) frei quellen, das heißt es wird am freien Quellen nahezu nicht gehindert. Die SAP-Partikel wirken nach dem Aufquellen wie ein "Gleitmittel" beziehungsweise die Flüssigkeitsspeicherschicht insgesamt wie eine Gleitschicht zwischen der Flüssigkeitsaufnahmeschicht und der Flüssigkeitsverteilungsschicht. Wie sich deutlich in den Versuchsnummern 16, 17 und 18 zeigt, wirkt sich die längere Quellzeit der absorbierenden Partikel reduzierend auf die Scherfestigkeitswerte aus.

Die Zugversuche mit den Nummern 19 bis 27 zeigen die Zugversuche zur Bestimmung der Scherfestigkeitswerte für die erfindungsgemäß ausgebildete Probe B. Die Proben B wurden mit Sprühkleber besprüht und mit Klebeband an den Außenlagen fixiert. Die Proben B weisen herstellungsbedingt eine dickere Flüssigkeitsspeicherschicht, das heißt eine dickere mittlere Schicht auf, die ebenfalls keine Bikomponentenfasern enthält. Die dickere Flüssigkeitsspeicherschicht bedingt ein leichteres Verschieben der Lagenstruktur in trockenem Zustand, was sich in der Tabelle 5 in den Zugversuchen mit den Nummern 19 bis 21 wiederfindet. Die bereits sehr geringe Scherfestigkeit der Probe B in trockenem Zustand wird durch eine Befeuchtung noch einmal reduziert und weist nach zehnminütiger Befeuchtung maximale Zugfestigkeiten von bis zu unter 3 N auf. Wirkt sich einerseits der Aufbau und insbesondere die dickere Flüssigkeitsspeicherschicht positiv auf eine Beweglichkeit der Lagenstruktur aus, so wird die Beweglichkeit durch die Befeuchtung der absorbierenden Partikel und deren "Gleitwirkung" nach einem nahezu ungehinderten Quellen der Lagenstruktur weiter gesteigert.

Die Zugversuche der ersten Vergleichsprobe MT 410.104 sind in den Zugversuchen mit den Nummern 28 bis 36 wiedergegeben. Zu erkennen ist deutlich, dass ausgehend von einer hohen Scherfestigkeit in trockenem Zustand von deutlich über 30 N die Scherfestigkeit in nassem Zustand nur geringfügig abfällt. Weist die Vergleichsprobe gemäß dem Zugversuch Nummer 28 eine Scherfestigkeit von F max$_{28}$ = 30,09 N auf, so fällt dieser Wert nach einminütiger Benässung der Probe lediglich auf F max$_{31}$ = 19,40 N ab. Die Vergleichsprobe MT 410.104 zeigt somit im trockenen sowie auch in nassem Zustand eine hohe Widerstandskraft gegen eine Scherbeanspruchung. Die Lagen der Struktur können somit nur unter Aufbringung hoher Kräfte gegeneinander verschoben werden.

Die Zugversuche gemäß den Nummern 37 bis 45 zeigen die Vergleichsproben VE 500.200 im trockenen und nassen Zustand und die Ergebnisse dieser Versuche. Im trockenen Zustand weist die Vergleichsprobe, wie beispielsweise im Zugversuch Nummer 37 festgehalten eine maximale Zugkraft von $F\,max_{37} = 50{,}73\,N$ auf. Die Scherfestigkeitswerte fallen stark bis zum Verlust der Integrität im nassen Zustand ab. Die Vergleichsprobe weist keine Schmelzklebefasern zur Nassbindung auf, so dass im nassen Zustand ein signifikanter Abfall der Scherfestigkeitswerte erfolgt. Dieser signifikante Abfall auf ein Achtel der ursprünglichen Scherfestigkeit wurde in den Zugversuchen Nummer 6 und 7, hier ist nass Beträufeln mit 10ml/g, bestätigt. Eine Nachmessung, hier mit komplettem Eintauchen in 0,9 %-iger NaCl-Lösung für 1 min und 10min, bestätigt den starken Abfall der Nass-Scherfestigkeitswerte, die Integrität als Lagenzusammenhalt ist nicht mehr gegeben, wie in aus den Versuchen 40 bis 45 ersichtlich.

Insbesondere zeigen die Messwerte aus der Tabelle 5 auch folgenden Zusammenhang: mit dem vorgeschlagenen Konzept gelingt es, eine Integrität der Lagenstruktur zu erzielen, die zum Einen im trockenen Zustand so vorliegt, dass die Lagenstruktur leicht biegbar und flexibel ist und sich dadurch gut an eine Kontur anpassen kann. Insbesondere ist diese Struktur nicht steif, wie es bei Lagenstrukturen ansonsten der Fall sein kann, die über eine hohe Festigkeit verfügen. Zum Anderen ist aber im nassen Zustand, d.h. bei eigentlieher Nutzung der Lagenstruktur durch Flüssigkeitsaufnahme, die Integrität der Lagen-struktur weiterhin noch so ausreichend vorhanden, dass diese nicht zerfällt. Dieses gilt insbesondere auch bei längerer Einwirkung von Flüssigkeit. So kann insbesondere ein auch unter Flüssigkeitseinwirkung weiterhin flexible Lagenstruktur geschaffen sein, die nicht versteift.

Beispielsweise haben sich die folgenden Ergebnisse als vorteilhaft bei der Auslegung einer vorgeschlagenen Lagenstruktur erwiesen:
So kann eine Ausgestaltung vorsehen, dass eine absorbierende Struktur ein Verhältnis einer Scherfestigkeit einer Probe, mit einer Breite von 25 mm und mit einer freien Einspannlänge von 200 mm, von nassen zu einem trockenen Zustand in einem Bereich von 1 zu 1,2, vorzugsweise 1 zu 2 bis zu einem Verhältnis von 1 zu 4 hat, wobei die absorbierende Struktur eine Minute in einer 0,9 %-ige NaCl-Lösung befeuchtet wurde.

Eine weitere Ausgestaltung kann vorsehen, dass die absorbierende Struktur ein Verhältnis einer Scherfestigkeit einer Probe, mit einer Breite von 25 mm und mit einer freien Einspannlänge von 200 mm, von nassen zu einem trockenen Zustand in einem Bereich von 1 zu 1,5 bis zu einem Verhältnis von 1 zu 5,33 hat, wobei die absorbierende Struktur zehn Minuten in einer 0,9 %-ige NaCl-Lösung befeuchtet wurde.

Des Weiteren kann auch vorgesehen sein, dass die Lagenstruktur im trockenen Zustand eine Reißfestigkeit von 6,8 bis 16 N/25mm und im Nasszustand eine Reißfestigkeit von 3 bis 6 N/25mm aufweist.

Auch besteht die Möglichkeit, dass die absorbierende Struktur in einem nassen Zustand eine Scherfestigkeit in einem Bereich von 3 N/25mm bis 6N/25mm aufweist. Gerade dieser Bereich ermöglicht, dass eine Flexibilität bei gleichzeitiger Integrität der Lagenstruktur erhalten bleibt. Gemäß einer Weiterbildung kann vorgesehen sein, dass die absorbierende Struktur im trockenen Zustand eine Scherfestigkeit in einem Bereich aufweist, die zwischen 5 und 7 N/25 mm liegt. Eine weitere Ausgestaltung sieht vor, dass die absorbierende Struktur im trockenen Zustand eine Scherfestigkeit von 11 und 17 N/25 mm aufweist.

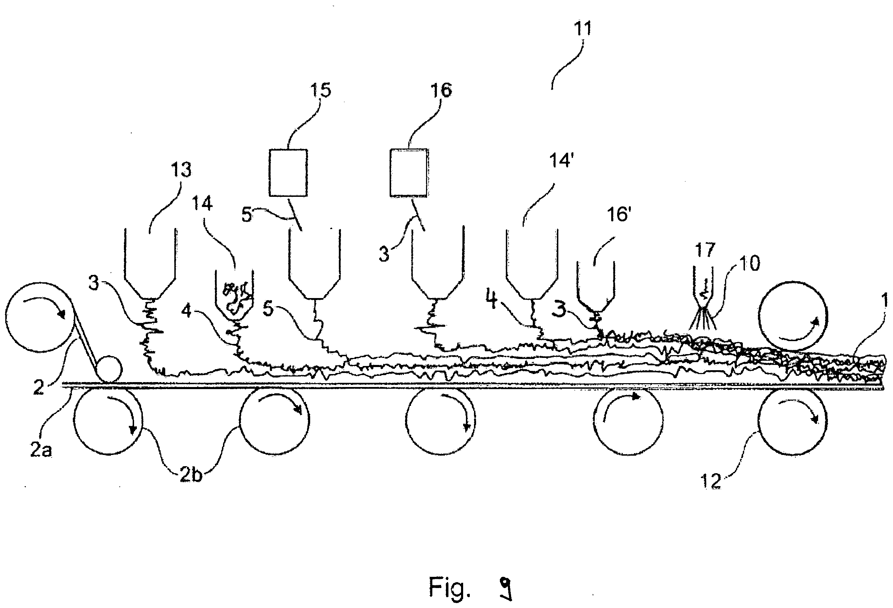

Fig.9 zeigt eine schematische Darstellung einer weiteren möglichen Ausgestaltung einer Vorrichtung 11 zur Herstellung einer absorbierenden Struktur 1. Es wird ein Inlineverfahren dargestellt mit einer Abwickelvorrichtung zur Bereitstellung einer Trägerlage als eine erste Schicht aus einem Airlaid-Material 2, welche auf ein Siebband 2a mit Antriebsrollen 2b aufgebracht wird. Weiterhin ist eine erste Formungseinrichtung 13 zum Ablegen von Fluffpulp 3 vorgesehen, eine Formungseinrichtung 14 zur Bereitstellung eines Absorbers, zum Beispiel SAP-Partikel 4, eine zusätzliche Formungseinrichtung 15 zur Bereitstellung von Bindefasern 5, zur Bildung einer zweiten Schicht 6, welche auf die Trägerlage 2 aufgebracht wird. Fluffpulp 3 und Absorber 4 werden durch die Formungseinrichtungen 16 und 14' bereit gestellt, um eine dritte Schicht 8 zu formen, welche auf die zweite Schicht 6 aufgebracht wird. Eine Formungseinrichtung 16' zur Bereitstellung von Fluffpulp 3 und eine Vorrichtung 17 zur Bereitstellung eines Bindemittels 10 werden eingesetzt, um eine vierte Schicht 9 zu bilden, welche auf die dritte Schicht aufgebracht wird. Ein Bindemittel 10, beispielsweise ein Latexbinder, wird mittels der Vorrichtung 17 auf die vierte Schicht 9, zum Beispiel durch Anwendungssprühen oder Rakelstreichen, aufzumindest eine Außenfläche der Schichten aufgebracht. Die Lagenstruktur kann nach dem Auftragen des Bindematerials durch Hitze aktiviert werden. Dieses kann aber mittels einer Beheizungseinrichtung erfolgen. Die Beheizungseinrichtung nutzt bevorzugt Strahlungswärme. Beispielswiese kann die Beheizungseinrichtung als Durchlaufstation gestaltet sein. Dabei oder danach wird die Lagenstruktur durch eine Vorrichtung 12 geleitet, beispielsweise einen Kalander, der einen Kalanderspalt enthält, in welchem die Lagenstruktur zusammengepresst wird. Die Vorrichtung 12 ist beispielsweise ein Kalander mit einer Anordnung leichtgängiger Rollen, kann jedoch auch ein Infrarot-Heizgerät, ein Ofenabschnitt oder ein anderer Heizkörper zur Aktivierung der Bindefasern sein, der die Lagen mit dem Bindematerial verklebt, sowie auch jeweils miteinander.

Aufgrund der Tests hat sich beispielsweise ergeben, dass eine Lagenstruktur ähnlich zu den oben beschriebenen Proben A und B vorzugsweise eine Verteilung an Schichtanteilen in Bezug auf das Gesamtgewicht der Lagenstruktur aufweist, welches wie folgt ist:

Schicht 1:    28 bis 39 Gew.-%
Schicht 2:    42 bis 55 Gew.-%
Schicht 3:    8,5 bis 16,5 Gew.-%

Hierbei kann eine Tissue-Lage zumindest auf einer Seite, insbesondere wie oben beschrieben, vorgesehen sein. Bevorzugt wird ein wetlaid Paper Tissue eingesetzt. Ist eine Tissue-Lage vorgesehen, weist diese beispielsweise einen Anteil am Gesamtgewicht der Lagenstruktur in einem Bereich von vorzugsweise 2,7 bis 5,5 Gew-% auf.

Besonders bevorzugt ist es, wenn keine der Außenlagen der Lagenstrukturen prägebondiert ist. So kann beispielweise vorgesehen sein, dass eine Wärmeeinwirkung vorhanden ist, die gleichmäßig vollflächig aufgebracht wird. Weiterhin ist es gemäß einer Ausgestaltung bevorzugt, dass für die Außenlagen nur ein Dispersionsbindemittel eingesetzt wird, insbesondere unter Verwendung von Latex. Bevorzugt wird ein Dispersionskleber eingesetzt, wie er für die Proben A und B beschrieben ist. Bevorzugt wird eine Dispersion benutzt, bei der Latex mit einem Anteil an der Dispersion bevorzugt in einem Bereich zwischen 11 bis 19,5 Gew-% genutzt wird. Des weiteren ist es bevorzugt, wenn das Dispersionsklebemittel, insbesondere das Latex bzw. EVA-Latex, im Trockenzustand der Lagenstruktur, d.h. nach Verdunstung der im Dispersionsmittel vorhandenen Flüssigkeit, in einem Bereich zwischen 1 Gew-% und 1,8 Gew-% jeweils auf jeder Seite der Lagenstruktur vorliegt.

Des Weiteren hat sich gezeigt, dass auch Ausgestaltungen ermöglicht werden, bei denen auf eine die Tissuelage verzichtet und eine strukturelle Integrität der jeweiligen Außenlagen durch das Dispersionsklebemittel im Zusammenwirken mit den Fasern der jeweiligen Außenlage der Lagenstruktur erfolgt. Insbesondere haben die Nasstests gezeigt, dass eine Integrität der Lagenstruktur auch mit einem derartigen Aufbau ausreichend vorhanden ist.

Insbesondere können mit den vorgeschlagenen Anteilen auch die oben beschriebenen Bereiche an Scherfestigkeit erzielt werden.

Tabelle 1: Rohstoffe und Zusammensetzung der Probe A (VH460.103)

| | | TRADE NAME | DESCRIPTION USE | CHEMICAL NAME | % BY WEIGHT | CAS REGISTRY # | COMPONENT SUPPLIER |
|---|---|---|---|---|---|---|---|
| | 1 | GP 4881 or | pulp, fibres | cellulose | 22 | 65996-61-4 | Georgia Pacific |
| | | NB 416 | | | | | Weyerhaeuser |
| | | | | | | | |
| | 2 | Biofluff TDR | pulp, fibres | cellulose | 21 | 65996-61-4 | Tembec Tartas |
| | | | | | | | |
| | 3 | Favor Z 3269 | superabsorber | poly acrylic acid. partly neutralised | 45 | 9003-04-7 | Evonik |
| | | | | | | | |
| | 4 | Vinnapas 192 or | polymer dispersion | ethylene vinyl acetate copolymer | 3 | 24937-78-8 | Wacker Polymers |
| | | Elite Ultra soft | | | | | Celanese |
| | | | | | | | |
| | 5 | T 255 | bicomponent fibres | polyethylene | 5 | 26221-73-8 | Trevira |
| | | | | polyethylene terephthalate | | 25038-59-9 | |
| | | | | | | | |
| | 6 | 3008 or | cellulose tissue | cellulose | 4 | 65996-61-4 | Cellu Tissue |
| | | KB 1800 | | | | | Swedish Tissue |
| | 7 | | | | | | |

(fortgesetzt)

| | | TRADE NAME | DESCRIPTION USE | CHEMICAL NAME | % BY WEIGHT | CAS REGISTRY # | COMPONENT SUPPLIER |
|---|---|---|---|---|---|---|---|
| | | | | | | | |
| | 8 | | | | | | |
| | | | | | | | |
| | 9 | | | | | | |
| | | | | | | | |
| | 10 | | | | | | |
| | | | | | | | |

Tabelle 2: Rohstoffe und Zusammensetzung der Probe B (VH600.101)

| | TRADE NAME | DESCRIPTION USE | CHEMICAL NAME | % BY WEIGHT | CAS REGISTRY # | COMPONENT SUPPLIER |
|---|---|---|---|---|---|---|
| 1 | GP 4881 or | pulp, fibres | cellulose | 24 | 65996-61-4 | Georgia Pacific |
| | NB 416 | | | | | Weyerhaeuser |
| | | | | | | |
| 2 | Biofluff TDR | pulp, fibres | cellulose | 20 | 65996-61-4 | Tembec Tartas |
| | | | | | | |
| 3 | Favor Z 3269 | superabsorber | poly acrylic acid, partly neutralised | 45 | 9003-04-7 | Evonik |
| | | | | | | |
| 4 | Vinnapas 192 or | polymer dispersion | ethylene vinyl acetate copolymer | 3 | 24937-78-8 | Wacker Polymers |
| | Elite Ultra soft | | | | | Celanese |
| | | | | | | |
| 5 | T 255 | bicomponent fibres | polyethylene | 5 | 26221-73-8 | Trevira |
| | | | polyethylene terephthalate | | 25038-59-9 | |
| | | | | | | |
| 6 | 3008 or | cellulose tissue | cellulose | 3 | 65996-61-4 | Cellu Tissue |
| | KB 1800 | | | | | Swedish Tissue |
| 7 | | | | | | |
| | | | | | | |
| 8 | | | | | | |
| | | | | | | |
| 9 | | | | | | |
| | | | | | | |
| 10 | | | | | | |

(fortgesetzt)

|  | TRADE NAME | DESCRIPTION USE | CHEMICAL NAME | % BY WEIGHT | CAS REGISTRY # | COMPONENT SUPPLIER |
|---|---|---|---|---|---|---|
|  |  |  |  |  |  |  |

**Tabelle 3**

| Messung der Schichtdicke | | | | | | | | |
|---|---|---|---|---|---|---|---|---|
| Testmethode WSP 120.6 | | | | | | | | |
|  | | | | | | | | |
| Produktcode | Position | GSM | Dicke (bei 0,5kPa) | | Dicke (bei 0,5kPa) | | Dicke (bei 0,5kPa) | |
|  | Einheit | g/m² | mm | | mm | | mm | |
|  | | | trocken | | 10g/g 0,9% NaCl-Lsg. | | freie Absorption lmin/2min abtropfen | |
|  | | | | | | | | |
| **VH600.101** | A4 | 631,01 | 7,55 | | | | | |
|  | | | | | | | | |
| 7cmx7cm samples | 1 | | 5,93 | | 9,25 | | | |
|  | 2 | | 6,10 | | 8,16 | | | |
|  | 3 | | 5,85 | | 8,13 | | | |
|  | 4 | | 6,12 | | 8,46 | | | |
|  | 5 | | 5,76 | | 8,27 | | | |
|  | 6 | | 5,75 | | | | 11 | |
|  | 7 | | 7,29 | | | | 11 | |
|  | 8 | | 7,45 | | | | 12 | |
|  | 9 | | 7,59 | | | | 12 | |
|  | 10 | | 7,69 | | | | 12 | |
|  | | | | | | | | |
|  | **avg** | | 6,55 | | 8,45 | | 11,6 | |
|  | | | | | | | | |
| **VH460.103** | A4 | 468,18 | 5,09 | | | | | |
|  | | | | | | | | |
| 7cmx7cm samples | 1 | | 5,06 | | 6,20 | | | |
|  | 2 | | 4,85 | | 6,03 | | | |
|  | 3 | | 4,93 | | 5,80 | | | |
|  | 4 | | 4,85 | | 5,48 | | | |
|  | 5 | | 4,90 | | 5,64 | | | |
|  | 6 | | 5,05 | | | | 9,79 | |
|  | 7 | | 5,13 | | | | 9,69 | |
|  | 8 | | 5,07 | | | | 9,81 | |

(fortgesetzt)

| Produktcode | Position | GSM | Dicke (bei 0,5kPa) | | Dicke (bei 0,5kPa) | | Dicke (bei 0,5kPa) | | |
|---|---|---|---|---|---|---|---|---|---|
| | Einheit | g/m² | mm | | mm | | mm | | |
| | | | trocken | | 10g/g 0,9% NaCl-Lsg. | | freie Absorption lmin/2min abtropfen | | |
| | 9 | | 5,14 | | | | 10,20 | | |
| | 10 | | 5,06 | | | | 10,18 | | |
| | | | | | | | | | |
| | avg | | 5,00 | | 5,83 | | 9,93 | | |
| **MT410.104** | A4 | 420,01 | 5,48 | | | | | | |
| | | | | | | | | | |
| 7cmx7cm samples | 1 | | 5,30 | | 5,61 | | | | |
| | 2 | | 5,40 | | 5,56 | | | | |
| | 3 | | 5,28 | | 5,55 | | | | |
| | 4 | | 5,34 | | 5,52 | | | | |
| | 5 | | 5,47 | | 5,54 | | | | |
| | 6 | | 5,41 | | | | 7,00 | | |
| | 7 | | 5,50 | | | | 7,17 | | |
| | 8 | | 5,50 | | | | 6,97 | | |
| | 9 | | 5,43 | | | | 7,08 | | |
| | 10 | | 5,42 | | | | 7,20 | | |
| | | | | | | | | | |
| | **avg** | | 5,41 | | 5,56 | | 7,08 | | |
| | | | | | | | | | |
| **VE500.200** | A4 | 473,84 | 1,59 | | | | | | |
| | | | | | | | | | |
| 7cmx7cm samples | 1 | | 1,67 | | 4,67 | | | | |
| | 2 | | 1,64 | | 4,38 | | | | |
| | 3 | | 1,58 | | 4,51 | | | | |
| | 4 | | 1,58 | | 3,66 | | | | |
| | 5 | | 1,59 | | 4,85 | | | | |
| | 6 | | 1,60 | | | | 7,34 | | |
| | 7 | | 1,60 | | | | 7,24 | | |
| | 8 | | 1,56 | | | | 7,52 | | |
| | 9 | | 1,59 | | | | 6,93 | | |
| | 10 | | 1,55 | | | | 6,60 | | |
| | avg | | 1,60 | | 4,41 | | 7,13 | | |

**Tabelle 4**

| Produktcode | Position | GSM | Beugelänge | Steife* 90.5 | Beugelänge | Steife* 90.5 | RF* 110.4 Opt.B | RF* 110.4 Opt.B | Bindung* 401.0 | Bindung* 401.0 |
|---|---|---|---|---|---|---|---|---|---|---|
| WSP-Methode | | | | | | | | | | |
| Einheit | | g/m² | cm | mN*cm | cm | mN*cm | N | | N | |
| | | | trocken | trocken | naß | naß | trocken | naß | trocken | naß |
| VH600.101 | 1 | 622,68 | 10,15 | 651,12 | 3,61 | 29,29 | 21,99 | 6,10 | 0,213 | 0,128 |
| | 2 | 646,35 | 10,13 | 671,89 | 3,65 | 31,43 | 25,40 | 6,57 | 0,142 | 0,148 |
| | 3 | 611,18 | 9,45 | 515,78 | 3,58 | 28,04 | 24,59 | 6,16 | 0,139 | 0,122 |
| | Mittelwert | 626,74 | | 612,93 | | 29,59 | 23,99 | 6,28 | 0,165 | 0,133 |
| VH460.103 | 1 | 477,34 | 8,58 | 301,50 | 2,99 | 12,76 | 23,69 | 4,37 | 0,143 | 0,145 |
| | 2 | 477,18 | 7,98 | 242,49 | 2,79 | 10,36 | 23,45 | 4,00 | 0,109 | 0,109 |
| | 3 | 491,84 | 8,13 | 264,30 | 2,94 | 12,50 | 22,13 | 4,70 | 0,114 | 0,114 |
| | Mittelwert | 482,12 | | 269,43 | | 11,87 | 23,09 | 4,36 | 0,122 | 0,123 |
| MT410.104 | 1 | 432,84 | 11,68 | 689,69 | 3,55 | 19,36 | 20,92 | 8,54 | 0,799 | 0,344 |
| | 2 | 424,18 | 11,58 | 658,68 | 3,31 | 15,38 | 17,35 | 8,14 | 0,565 | 0,342 |
| | 3 | 446,68 | 11,53 | 684,67 | 3,39 | 17,40 | 23,15 | 9,85 | 0,426 | 0,453 |
| | Mittelwert | 434,57 | | 677,68 | | 17,38 | 20,47 | 8,84 | 0,597 | 0,380 |
| VE500.200 | 1 | 484,01 | 7,15 | 176,92 | 2,91 | 11,93 | 13,54 | 2,82 | 0,460 | 0,034 |
| | 2 | 452,84 | 6,49 | 123,79 | 2,98 | 11,98 | 10,48 | 2,36 | 0,553 | 0,056 |
| | 3 | 436,01 | 6,86 | 140,76 | 2,96 | 11,31 | 11,60 | 2,48 | 0,486 | 0,077 |
| | Mittelwert | 457,62 | | 147,15 | | 11,74 | 11,87 | 2,55 | 0,500 | 0,056 |

naß= (10g/g 0,9%NaCl)

\* schmale Streifen (1inch = 25mm)

bei VH600.101 40ml

RF=Reissfestigkeit, 200mm Klammerabstand, Geschwindigkeit 100mm/min

(fortgesetzt)

| Produktcode | Position | GSM | Beugelänge | Steife* | Beugelänge | Steife* | RF * | | Bindung * | |
|---|---|---|---|---|---|---|---|---|---|---|
| | WSP-Methode | | | 90.5 | | 90.5 | 110.4 Opt.B | 110.4 Opt.B | 401.0 | 401.0 |
| | Einheit | g/m$^2$ | cm | mN*cm | cm | mN*cm | N | | N | |
| | | | | übrige Produkte 30ml | | | | | | |
| Absorption in g/g | | 0,9%ige Kochsalzlösung | | | | | | | | |
| WSP 10.1 | | 1Min. Tauchen | | | | | | | | |
| | | 2Min.hängen | | | | | | | | |
| | | | | | | | | | | * Teebeuteltest |

| Produktcode | Position | | | | | Mittelwert | | Methode Concert | | ABS/Total* |
|---|---|---|---|---|---|---|---|---|---|---|
| | 1 | 2 | 3 | 4 | 5 | g/g | | 10.Min.tauchen/10sec.tro. | | g/g |
| | | | | | | | | | | |
| VH600.101 | 17,75 | 17,35 | 17,36 | 18,06 | 17,63 | 17,63 | | 25,98 | | 30,12 |
| VH460.103 | 17,23 | 18,11 | 17,29 | 17,51 | 17,74 | 17,58 | | 27,20 | | 33,68 |
| MT410.104 | 15,44 | 14,97 | 15,75 | 15,33 | 14,83 | 15,26 | | 18,75 | | 20,77 |
| VE500.200 | 12,37 | 12,97 | 12,68 | 12,98 | 11,37 | 12,47 | | 18,94 | | 24,47 |
| | | | | | | | | | | |
| | Reißfestigkeit | in N/25mm | | | | | | | | |
| | | | | | | | | | | |
| | VH600.101 | komplett | 16,93 | | | | | | | |
| | | | 19,87 | | | | | | | |
| | | | 16,69 | | | | | | | |
| | VH600.101 | Tissue | 15,02 | | | | | | | |
| | | | 14,89 | | | | | | | |
| | | | 13,65 | | | | | | | |
| | VH600.101 | obere Seite | 5,05 | | | | | | | |
| | | | 5,63 | | | | | | | |

EP 2 528 567 B1

EP 2 528 567 B1

(fortgesetzt)

| Produktcode | Position | | | | | Mittelwert | | Methode Concert | | ABS/Total* |
|---|---|---|---|---|---|---|---|---|---|---|
| WSP 10.1 | | 1Min. Tauchen | | | | | | | | |
| | | 2Min.hängen | | | | | | | | |
| | | | | | | | | | | * Teebeuteltest |
| | | | 4,71 | | | | | | | |
| | | | 4,64 | | | | | | | |

**Patentansprüche**

1. Absorbierende Struktur (1, 20) mit einer Folge an Schichten umfassend
   eine erste und eine zweite Außenoberfläche,
   eine erste flüssigkeitsspeichernde Schicht (6, 22) enthaltend Fluffpulp (3), Bindefasern (5) und superabsorbierendes Polymer (4),
   eine zweite flüssigkeitsspeichernde Schicht (9', 24) enthaltend Fluffpulp (3) und Bindefasern (5),
   und mindestens eine zwischen der ersten flüssigkeitsspeichernden Schicht (6, 22) und der zweiten flüssigkeitsspeichernden Schicht (9', 24) und im direkten Kontakt mit diesen angeordnete Flüssigkeitsspeicherschicht (8', 23),
   wobei die Schichten übereinanderliegen und eine Lagenstruktur bilden,
   wobei die zumindest eine Flüssigkeitsspeicherschicht (8', 23) ein Fluffpulp (3, 7), ein superabsorbierendes Polymer (4) und keine Bindemittel aufweist, und
   wobei wenigstens eine der ersten und zweiten Außenoberflächen eine Latex-basierte Bindemittelschicht (10) aufweist.

2. Absorbierende Struktur (1, 20) nach Anspruch 1, **dadurch gekennzeichnet, dass** die erste und zweite Außenoberfläche jeweils eine Latex-basierte Bindemittelschicht (10) aufweisen.

3. Absorbierende Struktur (1, 20) nach einem der vorhergehenden Ansprüche, **dadurch gekennzeichnet, dass** diese eine Paper Tissue Lage (2, 21) als erste Lage (2, 21), die erste flüssigkeitsspeichernde Schicht (6, 22) als zweite Lage (6, 22), die Flüssigkeitsspeicherschicht (8', 23) als dritte Lage (8', 23) und die zweite flüssigkeitsspeichernde Schicht (9', 24) als vierte Lage (9', 24) aufweist.

4. Absorbierende Struktur (1, 20) nach einem der vorhergehenden Ansprüche, **dadurch gekennzeichnet, dass** die absorbierende Struktur (1, 20) ein Verhältnis einer Scherfestigkeit einer Probe, mit einer Breite von 25 mm und mit einer freien Einspannlänge von 200 mm, von nassen zu einem trockenen Zustand in einem Bereich von 1 zu 1,2 bis zu einem Verhältnis von 1 zu 4 hat, wobei die absorbierende Struktur (1, 20) eine Minute in einer 0,9 %-igen NaCl-Lösung befeuchtet wurde und anschließend zwei Minuten lang senkrecht abtropfen gelassen wurde und die Scherfestigkeit gemessen wird nach der in der Beschreibung enthaltenen Messmethode.

5. Absorbierende Struktur (1, 20) nach einem der vorhergehenden Ansprüche, **dadurch gekennzeichnet, dass** die absorbierende Struktur (1, 20) ein Verhältnis einer Scherfestigkeit einer Probe, mit einer Breite von 25 mm und mit einer freien Einspannlänge von 200 mm, von nassen zu einem trockenen Zustand in einem Bereich von 1 zu 1,5 bis zu einem Verhältnis von 1 zu 5,33 hat, wobei die absorbierende Struktur zehn Minuten in einer 0,9 %-igen NaCl-Lösung befeuchtet wurde und anschließend für zehn Sekunden senkrecht abtropfen gelassen wurde und die Scherfestigkeit gemessen wird nach der in der Beschreibung enthaltenen Messmethode.

6. Absorbierende Struktur (1, 20) nach einem der vorhergehenden Ansprüche, **dadurch gekennzeichnet, dass** die Lagenstruktur im trockenen Zustand eine Reißfestigkeit von 6,8 bis 16 N/25mm und im Nasszustand eine Reißfestigkeit von 3 bis 6 N/25mm aufweist,
   wobei die absorbierende Struktur (1, 20) eine Minute in einer 0,9 %-igen NaCl-Lösung befeuchtet wurde und anschließend zwei Minuten lang senkrecht abtropfen gelassen wurde oder die absorbierende Struktur zehn Minuten in einer 0,9 %-igen NaCl-Lösung befeuchtet wurde und anschließend für zehn Sekunden senkrecht abtropfen gelassen wurde und
   wobei die Reißfestigkeit nach der Standard-Test-Methode WSP 110.4 (05), Option B unter Verwendung einer Zwick-Prüfmaschine mit einem Klammerabstand von 200 mm und einem Vortrieb von 100 mm/min bestimmt wurde nach der in der Beschreibung enthaltenen Messmethode.

7. Absorbierende Struktur (1, 20) nach einem der vorhergehenden Ansprüche, **dadurch gekennzeichnet, dass** die absorbierende Struktur (1, 20) in einem nassen Zustand eine Scherfestigkeit in einem Bereich von 3 N/25mm bis 6 N/25mm aufweist,
   wobei die absorbierende Struktur (1, 20) eine Minute in einer 0,9 %-igen NaCl-Lösung befeuchtet wurde und anschließend zwei Minuten lang senkrecht abtropfen gelassen wurde oder die absorbierende Struktur zehn Minuten in einer 0,9 %-igen NaCl-Lösung befeuchtet wurde und anschließend für zehn Sekunden senkrecht abtropfen gelassen wurde und
   wobei die Scherfestigkeit analog zur Standard-Test-Methode WSP 110.4 (05), Option B unter Verwendung einer Zwick-Prüfmaschine mit einem Klammerabstand von 200 mm und einem Vortrieb von 100 mm/min bestimmt wurde nach der in der Beschreibung enthaltenen Messmethode.

8. Verfahren zur Herstellung einer absorbierenden Struktur (1, 20) nach einem der Ansprüche 1 bis 7, umfassend mindestens die folgenden Schritte:

   - Ablegen von Fluffpulp (3), superabsorbierendem Polymer (4) und Bindefasern (5) als zweite Lage (6) auf einer Tragschicht (2),
   - Ablegen von Fluffpulp (3) und superabsorbierendem Polymer (4) auf der zweiten Lage (6) als dritte Lage (8'), wobei keine Bindefasern (5) abgelegt werden,
   - Ablegen einer vierten Lage (9') aus Fluffpulp (3) und Bindefasern (5) auf der dritten Lage (8'),
   - Aufbringen einer Latex-basierten Bindemittelschicht (10) auf mindestens eine Außenoberfläche der Lagen,
   - Zuführen der Lagen zu einem Kalander (12), der einen Kalanderspalt aufweist, und
   - Verdichtung der Lagen im Kalanderspalt.

9. Vorrichtung (11) zur Herstellung einer absorbierenden Struktur (1, 20) nach einem der Ansprüche 1 bis 7, aufweisend zumindest:

   - ein Siebband (2a) zur Ablage von Lagen (6, 8', 9') zur Ausbildung einer Lagenstruktur,
   - eine erste Formungseinrichtung, über welche zumindest Fluffpulp (3) superabsorbierendes Polymer (4) und Bindefasern (5) auf dem Siebband (2a) aufbringbar sind, um eine Lage (6) zu bilden,
   - eine zweite Formungseinrichtung, über die Fluffpulp (3) und superabsorbierendes Polymer (4) auf dem Siebband (2a) aufbringbar sind, um eine weitere Lage (8') zu bilden,
   - eine dritte Formungseinrichtung, über die Bindefasern (5) und Fluffpulp (3) als weitere Lage (9') auf dem Siebband (2a) aufbringbar sind,
   - zumindest eine Auftragsstation (17), über die eine Latex-basierte Bindemittelschicht (10) auf eine äußere Oberfläche der Lagenstruktur aufbringbar ist,
   - eine Verdichtungsstation (12) über welche die Lagenstruktur verdichtbar ist und

   wobei die zweite Formungseinrichtung so ausgestaltet ist, dass mittels der zweiten Formungseinrichtung kein Bindemittel zugeführt wird oder werden kann.

10. Vorrichtung (11) gemäß Anspruch 9, wobei die Verdichtungsstation (12) ein Kalander (12) ist.

11. Verwendung einer absorbierenden Struktur (1, 20) nach einem der Ansprüche 1 bis 7 als Wegwerfprodukt, beispielsweise für den Hygienebereich, bevorzugt in einem Hygieneprodukt.

**Claims**

1. An absorbing structure (1, 20) with a sequence of layers, comprising:

   a first and a second outer surface,
   a first liquid storing layer (6, 22) comprising fluff pulp (3), bonding fibers (5) and super absorbing polymer (4),
   a second liquid storing layer (9', 24) comprising fluff pulp (3) and bonding fibers (5),
   and at least one liquid storage layer (8', 23) arranged between the first liquid storing layer (6, 22) and the second liquid storing layer (9', 24) and in direct contact with these,
   wherein the layers are disposed on top of one another and form a ply structure,
   wherein the at least one fluid storage layer (8', 23) comprises a fluff pulp (3, 7), a super absorbing polymer (4) and no binder material, and
   wherein at least one of the first and second outer surfaces comprises a latex based binder material layer (10).

2. Absorbing structure (1, 20) according to claim 1, **characterized in that** the first and second outer surfaces each comprise a latex based binder material layer (10).

3. Absorbing structure (1, 20) according to one of the previous claims, **characterized in that** it comprises a paper tissue ply (2, 21) as a first ply (2, 21), the first liquid storing layer (6, 22) as second ply (6, 22), the liquid storage layer (8', 23) as third ply (8', 23) and the second liquid storing layer (9', 24) as fourth ply (9', 24).

4. Absorbing structure (1, 20) according to one of the previous claims, **characterized in that** the absorbing structure (1, 20) has a ratio of a sheer strength of a sample with a width of 25 mm and with a free clamping length of 200 mm from a wet condition to a dry condition in a range of 1 to 1.2 up to a ratio of 1 to 4, wherein the absorbing structure (1, 20) was humidified for one minute in a 0.9 % NaCl solution and subsequently allowed to drip off for two minutes vertically suspended and the sheer strength is measured according to the measuring method as included in the description.

5. Absorbing structure (1, 20) according to one of the previous claims, **characterized in that** the absorbing structure (1, 20) has a ratio of a sheer strength of a sample with a width of 25 mm and with a free clamping length of 200 mm from a wet condition to a dry condition in a range of 1 to 1.5 up to a ratio of 1 to 5.33, wherein the absorbing structure (1, 20) was humidified for ten minute in a 0.9 % NaCl solution and subsequently allowed to drip off for ten seconds vertically suspended and the sheer strength is measured according to the measuring method as included in the description

6. Absorbing structure (1, 20) according to one of the previous claims, **characterized in that** the ply structure in dry condition has a tear strength of 6.8 to 16 N/25mm and in wet condition has a tear strength of 3 to 6 N/25mm, wherein the absorbing structure (1, 20) was humidified for one minute in a 0.9 % NaCl solution and subsequently allowed to drip off for two minutes vertically suspended or the absorbing structure (1, 20) was humidified for ten minute in a 0.9 % NaCl solution and subsequently allowed to drip off for ten seconds vertically suspended, and wherein the tear strength was determined according to the standard test method WSP 110.4 (05), option B, using a Zwick test apparatus with a clamp distance of 200 mm and a feed of 100 mm/min according to the measuring method included in the description.

7. Absorbing structure (1, 20) according to one of the previous claims, **characterized in that** the absorbing structure (1, 20) in a wet condition has a sheer strength in a range of 3 N/25 mm to 6 N/25mm, wherein the absorbing structure (1, 20) was humidified for one minute in a 0.9 % NaCl solution and subsequently allowed to drip off for two minutes vertically suspended or the absorbing structure (1, 20) was humidified for ten minute in a 0.9 % NaCl solution and subsequently allowed to drip off for ten seconds vertically suspended, and wherein the sheer strength was determined analogously to the standard test method WSP 110.4 (05), option B, using a Zwick test apparatus with a clamp distance of 200 mm and a feed of 100 mm/min according to the measuring method included in the description.

8. Method for producing an absorbing structure (1, 20) according to one of the claims 1 to 7, comprising at least the following steps:

   - laying fluff pulp (3), super absorbing polymer (4) and bonding fibers (5) as a second ply (6) on a support layer (2),
   - laying fluff pulp (3) and super absorbing polymer (4) on the second ply (6) as a third ply (8'), wherein no bonding fibers (5) are laid down,
   - laying a fourth ply (9') made of fluff pulp (3) and bonding fibers (5) on the third ply (8'),
   - applying a latex based binder material layer (10) on at least one outer surface of the plies,
   - supplying the plies to a calender (12) which includes a calender gap, and
   - compressing the plies in the calender gap.

9. Device (11) for producing an absorbing structure (1, 20), according to one of the claims 1 to 7, comprising at least:

   - a sifting band (2a) for laying plies (6, 8', 9') for forming a ply structure,
   - a first forming device through which at least fluff pulp (3), super absorbing polymer (4), and bonding fibers (5) are applicable to the sifting band (2a) for forming a ply (6),
   - a second forming device through which fluff pulp (3) and super absorbing polymer (4) are applicable to the sifting band (2a) for forming an additional ply (8'),
   - a third forming device through which bonding fibers (5) and fluff pulp (3) are applicable to the sifting band (2a) as an additional ply (9'),
   - at least one application station (17) through which a latex based binder material layer (10) is applicable on an outer surface of the ply structure,
   - a compressing station (12) through which the ply structure is compressible and

   wherein the second forming device is configured such that no binder material is supplied or can be supplied by the second forming device.

**10.** Device (11) according to claim 9, wherein the compressing station (12) is a calender (12).

**11.** Use of an absorbing structure (1, 20) according to one of the claims 1 to 7 as disposable product, for example, for a hygiene application, preferably in a hygiene product.

## Revendications

**1.** Structure absorbante (1, 20) avec une séquence de couches comprenant :

une première et une deuxième surface extérieure,
une première couche d'accumulation de liquide (6, 22) contenant de la pâte bouffante (3), des fibres de liaison (5) et un polymère superabsorbant (4),
une deuxième couche d'accumulation de liquide (9', 24) contenant de la pâte bouffante (3) et des fibres de liaison (5), et
au moins une couche d'accumulation de liquide (8', 23) disposée entre la première couche d'accumulation de liquide (6, 22) et la deuxième couche d'accumulation de liquide (9', 24) et en contact direct avec elles,
les couches se trouvant l'une au-dessus de l'autre et formant une structure en couches,
la au moins une couche d'accumulation de liquide (8', 23) présentant une pâte bouffante (3, 7), un polymère superabsorbant (4) et aucun liant, et
au moins l'une des première et deuxième surfaces externes présente une couche de liant à base de latex (10).

**2.** Structure absorbante (1, 20) selon la revendication 1, **caractérisée en ce que** les première et deuxième surfaces extérieures comportent chacune une couche de liant à base de latex (10).

**3.** Structure absorbante (1, 20) selon l'une des revendications précédentes, **caractérisée en ce qu'**elle présente une couche de papier mousseline (2, 21) comme première couche (2, 21), la première couche d'accumulation de liquide (6, 22) comme deuxième couche (6, 22), la couche d'accumulation de liquide (8', 23) comme troisième couche (8', 23) et la deuxième couche d'accumulation de liquide (9', 24) comme quatrième couche (9', 24).

**4.** Structure absorbante (1, 20) selon l'une des revendications précédentes, **caractérisée en ce que** la structure absorbante (1, 20) présente un rapport de résistance au cisaillement d'un échantillon d'une largeur de 25 mm et d'une longueur de serrage libre de 200 mm, d'un état humide à un état sec dans une plage de 1 à 1,2 jusqu'à un rapport de 1 à 4, la structure absorbante (1, 20) étant humidifiée dans une solution de NaCl à 0,9% pendant une minute, puis est laissée s'égoutter verticalement pendant deux minutes et la résistance au cisaillement est mesurée selon la méthode de mesure contenue dans la description.

**5.** Structure absorbante (1, 20) selon l'une des revendications précédentes, **caractérisée en ce que** la structure absorbante (1, 20) présente un rapport de résistance au cisaillement d'un échantillon, d'une largeur de 25 mm et d'une longueur de serrage libre de 200 mm, de humide à sec dans une plage de 1 à 1,5 à un rapport de 1 à 5,33, la structure absorbante étant humidifiée dans une solution de NaCl à 0,9% pendant dix minutes, puis est laissée pendant dix secondes s'égoutter verticalement et la résistance au cisaillement est mesurée selon la méthode de mesure contenue dans la description.

**6.** Structure absorbante (1, 20) selon l'une des revendications précédentes, **caractérisée en ce que** la structure en couches à l'état sec a une résistance à la déchirure de 6,8 à 16 N/25 mm et à l'état humide une résistance à la déchirure de 3 à 6 N/25mm
dans laquelle la structure absorbante (1, 20) est humidifiée dans une solution de NaCl à 0,9% pendant une minute, puis pendant deux minutes est laissée s'égoutter verticalement, ou la structure absorbante est humidifiée dans une solution de NaCl à 0,9% pendant dix minutes, puis pendant dix secondes est laissée s'égoutter verticalement, et la résistance à la déchirure est déterminée selon la méthode d'essai standard WSP 110.4 (05), option B en utilisant une machine d'essai Zwick avec un espacement des pinces de 200 mm et un avancement de 100 mm/min selon la méthode de mesure contenue dans la description.

**7.** Structure absorbante (1, 20) selon l'une des revendications précédentes, **caractérisée en ce que** la structure absorbante (1, 20) à l'état humide a une résistance au cisaillement comprise entre 3 N/25mm et 6 N/25mm,
dans laquelle la structure absorbante (1, 20) est humidifiée dans une solution de NaCl à 0,9% pendant une minute, puis pendant deux minutes est laissée s'égoutter verticalement ou la structure absorbante est humidifiée dans une solution de NaCl à 0,9% pendant dix minutes, puis pendant dix secondes est laissée s'égoutter verticalement, et la résistance au cisaillement est déterminée de façon analogue à la méthode d'essai standard WSP 110.4 (05), option B en utilisant une machine d'essai Zwick avec un espacement des pinces de 200 mm et un avancement de 100 mm/min selon la méthode de mesure contenue dans la description.

8.  Procédé de fabrication d'une structure absorbante (1, 20) selon l'une des revendications 1 à 7, comprenant au moins les étapes suivantes :

- dépôt de pâte bouffante (3), de polymère superabsorbant (4) et de fibres de liaison (5) en tant que deuxième couche (6) sur une couche de base (2),
- dépôt de pâte bouffante (3) et de polymère superabsorbant (4) sur la deuxième couche (6) en tant que troisième couche (8'), aucune fibre liante (5) n'étant déposée,
- dépôt d'une quatrième couche (9') de pâte bouffante (3) et de fibres de liaison (5) sur la troisième couche (8'),
- application d'une couche de liant à base de latex (10) sur au moins une surface externe des couches,
- acheminement des couches vers une calandre (12) qui a un espace de calandre,
et

compression des couches dans l'espace de calandre.

9.  Dispositif (11) de réalisation d'une structure absorbante (1, 20) selon l'une des revendications 1 à 7, comprenant au moins :

- un tapis de tamisage (2a) pour déposer des couches (6, 8', 9') pour former une structure en couches,
- un premier dispositif de mise en forme, par l'intermédiaire duquel au moins de la pâte bouffante (3), le polymère superabsorbant (4) et des fibres de liaison (5) peuvent être appliqués sur la bande de tamisage (2a) pour former une couche (6),
- un deuxième dispositif de mise en forme, par l'intermédiaire duquel la pâte bouffante (3) et le polymère superabsorbant (4) peuvent être appliqués sur la bande de tamisage (2a) pour former une couche supplémentaire (8'),
- un troisième dispositif de mise en forme, par l'intermédiaire duquel les fibres de liaison (5) et la pâte bouffante (3) peuvent être appliqués comme couche supplémentaire (9') sur la bande de tamisage (2a),
- au moins un poste d'application (17), par l'intermédiaire duquel une couche de liant à base de latex (10) peut être appliquée sur une surface externe de la structure en couches,
- un poste de compression (12), par l'intermédiaire duquel la structure en couches peut être comprimée, et

le deuxième dispositif de mise en forme étant conçu de manière qu'aucun liant n'est ou ne peut être fourni au moyen du deuxième dispositif de mise en forme.

10. Dispositif (11) selon la revendication 9, dans lequel le poste de compression (12) est une calandre (12).

11. Utilisation d'une structure absorbante (1, 20) selon l'une des revendications 1 à 7 comme produit jetable, par exemple pour le secteur de l'hygiène, de préférence dans un produit d'hygiène.

Fig. 1

Fig. 2

EP 2 528 567 B1

**Fig. 3**

EP 2 528 567 B1

Fig. 4

Probe A (VH460.103)

31

29

28

27

26

25

30

32

Fig. 5

EP 2 528 567 B1

Probe B (VH600.101)

Fig. 6

Fig. 7

| Nr | Probe | Fmax N | Dehnung % | Weg mm |
|----|-------|--------|-----------|--------|
| 1 | VH600.101 tr.mit Tape 4024 tesa | 6,82 | 4,58 | 9,19 |
| 2 | VH600.101 n.mit Tape,Airl.besprüht | 5,06 | 1,92 | 3,86 |
| 3 | VH600.101 n.Tape+Airl.besprüht | 5,65 | 2,52 | 5,06 |
| 4 | MT410.104 tr. mit Tape 4024 tesa | 34,94 | 5,60 | 11,22 |
| 5 | MT410.104 n.mit Tape/ Airlaid besprüht | 17,72 | 3,75 | 7,52 |
| 6 | VE500.200 tr.mit Tape 4024 tesa | 41,26 | 2,98 | 5,97 |
| 7 | VE500.200 n.mit Tape / Airlaid besprüht | 5,59 | 0,96 | 1,92 |
| 8 | VH460.103 tr. mit Tape 4024 tesa | 11,66 | 3,80 | 7,62 |
| 9 | VH460.103 n. mit Tape /Airlaid besprüht | 5,65 | 2,35 | 4,72 |
| 10 | VH460.103 mit Tape /Airlaid besprüht | 16,22 | 3,97 | 7,96 |
| 11 | VH460.103 mit Tape /Airlaid besprüht | 16,11 | 3,81 | 7,66 |
| 12 | VH460.103 mit Tape /Airlaid besprüht | 14,75 | 3,84 | 7,70 |
| 13 | VH460.103 mit T.+besprüht/1Min. nass | 5,16 | 3,55 | 7,14 |
| 14 | VH460.103 mit T.+besprüht/1Min. nass | 4,18 | 3,16 | 6,34 |
| 15 | VH460.103 mit T.+besprüht/1Min. nass | 4,92 | 2,72 | 5,46 |
| 16 | VH460.103 mit T.+besprüht/10Min. nass | 3,12 | 3,35 | 6,83 |
| 17 | VH460.103 mit T.+besprüht/10Min. nass | 3,87 | 4,48 | 9,03 |
| 18 | VH460.103 mit T.+besprüht/10Min. nass | 3,26 | 3,78 | 7,64 |
| 19 | VH600.101 mit T.+besprüht/ | 5,13 | 3,58 | 7,19 |
| 20 | VH600.101 mit T.+besprüht/ | 4,04 | 2,88 | 5,81 |
| 21 | VH600.101 mit T.+besprüht/ | 4,94 | 2,62 | 5,26 |
| 22 | VH600.101 mit T.+besprüht/1Min. nass | 3,75 | 2,81 | 5,68 |
| 23 | VH600.101 mit T.+besprüht/1Min. nass | 3,19 | 2,12 | 4,28 |
| 24 | VH600.101 mit T.+besprüht/1Min. nass | 4,21 | 3,21 | 6,48 |
| 25 | VH600.101 mit T.+besprüht/10Min. nass | 3,53 | 3,32 | 6,66 |
| 26 | VH600.101 mit T.+besprüht/10Min. nass | 2,84 | 3,23 | 6,49 |
| 27 | VH600.101 mit T.+besprüht/10Min. nass | 2,99 | 3,98 | 7,99 |
| 28 | MT410.104 mit T.+besprüht/ | 30,09 | 4,83 | 9,73 |
| 29 | MT410.104 mit T.+besprüht/ | 32,03 | 6,46 | 12,93 |
| 30 | MT410.104 mit T.+besprüht/ | 35,17 | 5,51 | 11,03 |
| 31 | MT410.104 mit T.+besprüht/1Min. nass | 19,40 | 3,29 | 6,59 |
| 32 | MT410.104 mit T.+besprüht/1Min. nass | 16,76 | 3,42 | 6,86 |
| 33 | MT410.104 mit T.+besprüht/1Min. nass | 19,22 | 3,69 | 7,41 |
| 34 | MT410.104 mit T.+besprüht/10Min. nass | 16,52 | 5,07 | 10,19 |
| 35 | MT410.104 mit T.+besprüht/10Min. nass | 17,69 | 3,98 | 7,98 |
| 36 | MT410.104 mit T.+besprüht/10Min. nass | 17,18 | 4,42 | 8,88 |
| 37 | VE500.200 mit T.+besprüht/ | 50,73 | 6,07 | 12,18 |
| 38 | VE500.200 mit T.+besprüht/ | 55,81 | 6,77 | 13,56 |
| 39 | VE500.200 mit T.+besprüht/ | 54,23 | 7,54 | 15,11 |
| 40 | VE500.200 mit T.+besprüht/1Min. nass | 2,40 | 1,06 | 2,13 |
| 41 | VE500.200 mit T.+besprüht/1Min. nass | 2,48 | 1,03 | 2,06 |
| 42 | VE500.200 mit T.+besprüht/1Min. nass | 1,55 | 0,79 | 1,58 |
| 43 | VE500.200 mit T.+besprüht/10Min. nass | 1,07 | 1,32 | 2,65 |
| 44 | VE500.200 mit T.+besprüht/10Min. nass | 1,26 | 2,16 | 4,33 |
| 45 | VE500.200 mit T.+besprüht/10Min. nass | 0,72 | 2,57 | 5,23 |

Tabelle 5: Zugversuch Bestimmung Scherfestigkeitswerte

Fig. 8

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1721036 A
- US 20020165509 A1
- EP 2140844 A1
- US 20040122394 A1
- EP 0810886 A
- DE 102004015686 A1
- DE 69821794
- DE 102004005417 A1
- DE 10232078 A1
- DE 10251137 A1
- DE 69808061
- DE 102004009556 A1
- DE 102004024551 B4
- DE 102004056154 A1
- DE 10327026 A1
- DE 19918343 A1
- WO 2005080655 A1
- WO 03034963 A2
- US 12657987 B
- DE 102010006228